# EUROPEAN PATENT SPECIFICATION

(11) **EP 2 707 501 B1**
(45) Date of publication and mention of the grant of the patent: **28.02.2018**
(21) Application number: 12720167.1
(22) Date of filing: 09.05.2012
(51) Int. Cl.: C12Q 1/68

(54) **MOLECULAR MARKERS IN PROSTATE CANCER**
MOLEKULARE MARKER BEI PROSTATAKARZINOM
MARQUEURS MOLÉCULAIRES DU CANCER DE LA PROSTATE

(30) Priority: 12.05.2011 WO PCT/EP2011/057716
(43) Date of publication of application: 19.03.2014
(73) Proprietor: MDxHealth Research B.V., 6525 GA Nijmegen (NL)
(72) Inventor: SMIT, Franciscus, Petrus, NL-6546 RN Nijmegen (NL)
(74) Representative: van Kooij, Adriaan
(86) International application number: PCT/EP2012/058502
(87) International publication number: WO 2012/152811

(56) References cited:
- WO-A1-2009/015229
- WO-A1-2009/042742
- WO-A1-2010/037735
- WO-A2-2004/060304
- MICHELLE R. DOWNES ET AL: "Urinary markers for prostate cancer", BJU INTERNATIONAL, vol. 99, no. 2, 1 February 2007 (2007-02-01), pages 263-268, XP055014164, ISSN: 1464-4096, DOI: 10.1111/j.1464-410X.2006.06610.x
- VAN GILS M P M Q ET AL: "Innovations in Serum and Urine Markers in Prostate Cancer", EUROPEAN UROLOGY, ELSEVIER BV, NL, vol. 48, no. 6, 1 December 2005 (2005-12-01), pages 1031-1041, XP027606458, ISSN: 0302-2838 [retrieved on 2005-12-01]
- TALESA VINCENZO N ET AL: "Diagnostic potential in prostate cancer of a panel of urinary molecular tumor markers", CANCER BIOMARKERS, IOS PRESS, AMSTERDAM, NL, vol. 5, no. 6, 1 January 2009 (2009-01-01) , pages 241-251, XP009148896, ISSN: 1574-0153
- Bussemakers et al.: "DD3: A New Prostate-specific Gene, highly Overexpressed in Prostate Cancer", , 1 December 1999 (1999-12-01), XP055030609, Retrieved from the Internet: URL:http://cancerres.aacrjournals.org/cont ent/59/23/5975.full.pdf [retrieved on 2012-06-21]
- YVES FRADET ET AL: "uPM3, a new molecular urine test for the detection of prostate cancer", UROLOGY, vol. 64, no. 2, 1 August 2004 (2004-08-01) , pages 311-315, XP055030611, ISSN: 0090-4295, DOI: 10.1016/j.urology.2004.03.052

## Description

The present invention relates to methods for diagnosing prostate cancer (PrCa).

In the Western male population, prostate cancer has become a major public health problem. In many developed countries it is not only the most commonly diagnosed malignancy, but it is the second leading cause of cancer related deaths in males as well. Because the incidence of prostate cancer increases with age, the number of newly diagnosed cases continues to rise as the life expectancy of the general population increases. In the United States, approximately 218,000 men, and in Europe approximately 382,000 men are newly diagnosed with prostate cancer every year.

Epidemiology studies show that prostate cancer is an indolent disease and that more men die with prostate cancer than from it. However, a significant fraction of the tumors behave aggressively and as a result approximately 32,000 American men and approximately 89,000 European men die from this disease on a yearly basis.

The high mortality rate is a consequence of the fact that there are no curative therapeutic options for metastatic prostate cancer. Androgen ablation is the treatment of choice in men with metastatic disease. Initially, 70 to 80% of the patients with advanced disease show response to the therapy, but with time the majority of the tumors will become androgen independent. As a result most patients will develop progressive disease.

Since there are no effective therapeutic options for advanced prostate cancer, early detection of this tumor is pivotal and can increase the curative success rate. Although the routine use of serum prostate-specific antigen (PSA) testing has undoubtedly increased prostate cancer detection, one of its main drawbacks has been the lack of specificity. Serum PSA is an excellent marker for prostatic diseases and even modest elevations almost always reflect a disease or perturbation of the prostate gland including benign prostatic hyperplasia (BPH) and prostatitis. Since the advent of frequent PSA testing over 20 years ago, the specificity of PSA for cancer has declined due to the selection of a large number of men who have elevated PSA due to non-cancer mechanisms. This results in a high negative biopsy rate.

Therefore, (non-invasive) molecular tests, that can accurately identify those men who have early stage, clinically localized prostate cancer and who would gain prolonged survival and quality of life from early radical intervention, are urgently needed. Molecular biomarkers identified in tissues can serve as target for new body fluid based molecular tests.

A suitable biomarker preferably fulfils the following criteria: 1) it must be reproducible (intra- and inter-institutional) and 2) it must have an impact on clinical management.

Further, for diagnostic purposes, it is important that the biomarkers are tested in terms of tissue-specificity and discrimination potential between prostate cancer, normal prostate and BPH. Furthermore, it can be expected that (multiple) biomarker-based assays enhance the specificity for cancer detection.

Considering the above, there is an urgent need for molecular prognostic biomarkers for predicting the biological behaviour of cancer and outcome.

For the identification of new candidate markers for prostate cancer, it is necessary to study expression patterns in malignant as well as non-malignant prostate tissues, preferably in relation to other medical data.

Recent developments in the field of molecular techniques have provided new tools that enabled the assessment of both genomic alterations and proteomic alterations in these samples in a comprehensive and rapid manner. These tools have led to the discovery of many new promising biomarkers for prostate cancer. These biomarkers may be instrumental in the development of new tests that have a high specificity in the diagnosis and prognosis of prostate cancer.

For instance, the identification of different chromosomal abnormalities like changes in chromosome number, translocations, deletions, rearrangements and duplications in cells can be studied using fluorescence in situ hybridization (FISH) analysis. Comparative genomic hybridization (CGH) is able to screen the entire genome for large changes in DNA sequence copy number or deletions larger than 10 mega-base pairs. Differential display analysis, serial analysis of gene expression (SAGE), oligonucleotide arrays and cDNA arrays characterize gene expression profiles. These techniques are often used combined with tissue microarray (TMA) for the identification of genes that play an important role in specific biological processes.

Since genetic alterations often lead to mutated or altered proteins, the signalling pathways of a cell may become affected. Eventually, this may lead to a growth-advantage or survival of a cancer cell. Proteomics study the identification of altered proteins in terms of structure, quantity, and post-translational modifications. Disease-related proteins can be directly sequenced and identified in intact whole tissue sections using the matrix-assisted laser desorption-ionization time-of-flight mass spectrometer (MALDI-TOF). Additionally, surface-enhanced laser desorption-ionization (SELDI)-TOF mass spectroscopy (MS) can provide a rapid protein expression profile from tissue cells and body fluids like serum or urine.

In the last years, these molecular tools have led to the identification of hundreds of genes that are believed to be relevant in the development of prostate cancer. Not only have these findings led to more insight in the initiation and progression of prostate cancer, but they have also shown that prostate cancer is a heterogeneous disease.

Several prostate tumors may occur in the prostate of a single patient due to the multifocal nature of the disease. Each of these tumors can show remarkable differences in gene expression and behaviour that are associated with varying prognoses. Therefore, in predicting the outcome of the disease it is more likely that a set of different markers will become clinically important.

Biomarkers can be classified into four different prostate cancer-specific events: genomic alterations, prostate cancer-specific biological processes, epigenetic modifications and genes uniquely expressed in prostate cancer.

One of the strongest epidemiological risk factors for prostate cancer is a positive family history. A study of 44,788 pairs of twins in Denmark, Sweden and Finland has shown that 42% of the prostate cancer cases were attributable to inheritance. Consistently higher risk for the disease has been observed in brothers of affected patients compared to the sons of the same patients. This has led to the hypothesis that there is an X-linked or recessive genetic component involved in the risk for prostate cancer.

Genome-wide scans in affected families implicated at least seven prostate cancer susceptibility loci, HPC1 (1q24), CAPB (1p36), PCAP (1q42), ELAC2 (17p11), HPC20 (20q13), 8p22-23 and HPCX (Xq27-28). Recently, three candidate hereditary prostate cancer genes have been mapped to these loci, HPC1/2'-5'-oligoadenylate dependent ribonuclease L (RNASEL) on chromosome 1q24-25, macrophage scavenger 1 gene (MSR1) located on chromosome 8p22-23, and HPC2/ELAC2 on chromosome 17p11.

It has been estimated that prostate cancer susceptibility genes probably account for only 10% of hereditary prostate cancer cases. Familial prostate cancers are most likely associated with shared environmental factors or more common genetic variants or polymorphisms. Since such variants may occur at high frequencies in the affected population, their impact on prostate cancer risk can be substantial.

Recently, polymorphisms in the genes coding for the androgen-receptor (AR), 5α-reductase type II (SRD5A2), CYP17, CYP3A, vitamin D receptor (VDR), PSA, GST-T1, GST-M1, GST-P1, insulin-like growth factor (IGF-I), and IGF binding protein 3 (IGFBP3) have been studied.

These studies were performed to establish whether these genes can predict the presence of prostate cancer in patients indicated for prostate biopsies due to PSA levels >3 ng/ml. No associations were found between AR, SRD5A2, CYP17, CYP3A4, VDR, GST-M1, GST-P1, and IGFBP3 genotypes and prostate cancer risk. Only GST-T1 and IGF-I polymorphisms were found to be modestly associated with prostate cancer risk.

Unlike the adenomatous polyposis coli (APC) gene in familial colon cancer, none of the mentioned prostate cancer susceptibility genes and loci is by itself responsible for the largest portion of prostate cancers.

Epidemiology studies support the idea that most prostate cancers can be attributed to factors as race, life-style, and diet. The role of gene mutations in known oncogenes and tumor suppressor genes is probably very small in primary prostate cancer. For instance, the frequency of p53 mutations in primary prostate cancer is reported to be low but have been observed in almost 50% of advanced prostate cancers.

Screening men for the presence of cancer-specific gene mutations or polymorphisms is time-consuming and costly. Moreover, it is very ineffective in the detection of primary prostate cancers in the general male population. Therefore, it cannot be applied as a prostate cancer screening test.

Mitochondrial DNA is present in approximately 1,000 to 10,000 copies per cell. Due to these quantities, mitochondrial DNA mutations have been used as target for the analysis of plasma and serum DNA from prostate cancer patients. Recently, mitochondrial DNA mutations were detected in three out of three prostate cancer patients who had the same mitochondrial DNA mutations in their primary tumor. Different urological tumor specimens have to be studied and larger patient groups are needed to define the overall diagnostic sensitivity of this method.

Critical alterations in gene expression can lead to the progression of prostate cancer. Microsatellite alterations, which are polymorphic repetitive DNA sequences, often appear as loss of heterozygosity (LOH) or as microsatellite instability. Defined microsatellite alterations are known in prostate cancer. The clinical utility so far is neglible. Whole genome- and SNP arrays are considered to be powerful discovery tools.

Alterations in DNA, without changing the order of bases in the sequence, often lead to changes in gene expression. These epigenetic modifications include changes such as DNA methylation and histone acetylation/deacetylation. Many gene promoters contain GC-rich regions also known as CpG islands. Abnormal methylation of CpG islands results in decreased transcription of the gene into mRNA.

Recently, it has been suggested that the DNA methylation status may be influenced in early life by environmental exposures, such as nutritional factors or stress, and that this leads to an increased risk for cancer in adults. Changes in DNA methylation patterns have been observed in many human tumors. For the detection of promoter hypermethylation a technique called methylation-specific PCR (MSP) is used. In contrast to microsatellite or LOH analysis, this technique requires a tumor to normal ratio of only 0.1-0.001%. This means that using this technique, hypermethylated alleles from tumor DNA can be detected in the presence of 10⁴ -10⁵ excess amounts of normal alleles.

Therefore, DNA methylation can serve as a useful marker in cancer detection. Recently, there have been many reports on hypermethylated genes in human prostate cancer. Two of these genes are RASSF1A and GSTP1.

Hypermethylation of RASSF1A (ras association domain family protein isoform A) is a common phenomenon in breast cancer, kidney cancer, liver cancer, lung cancer and prostate cancer. The growth of human cancer cells can be reduced when *RASSF1A* is re-expressed. This supports a role for *RASSF1A* as a tumor suppressor gene. Initially no *RASSF1A* hypermethylation was detected in normal prostate tissue. Recently, methylation of the *RASSF1A* gene was observed in both pre-malignant prostatic intra-epithelial neoplasms and benign prostatic epithelia. *RASSF1A* hypermethylation has been observed in 60-74% of prostate tumors and in 18.5% of BPH samples. Furthermore, the methylation frequency is clearly associated with high Gleason score and stage. These findings suggest that *RASSF1A* hypermethylation may distinguish the more aggressive tumors from the indolent ones.

The most described epigenetic alteration in prostate cancer is the hypermethylation of the Glutathione S-transferase P1 (GSTP1) promoter. GSTP1 belongs to the cellular protection system against toxic effects and as such this enzyme is involved in the detoxification of many xenobiotics.

GSTP1 hypermethylation has been reported in approximately 6% of the proliferative inflammatory atrophy (PIA) lesions and in 70% of the PIN lesions. It has been shown that some PIA lesions merge directly with PIN and early carcinoma lesions, although additional studies are necessary to confirm these findings. Hypermethylation of GSTP1 has been detected in more than 90% of prostate tumors, whereas no hypermethylation has been observed in BPH and normal prostate tissues.

Hypermethylation of the GSTP1 gene has been detected in 50% of ejaculates from prostate cancer patients but not in men with BPH. Due to the fact that ejaculates are not always easily obtained from prostate cancer patients, hypermethylation of GSTP1 was determined in urinary sediments obtained from prostate cancer patients after prostate massage. Cancer could be detected in 77% of these sediments.

Moreover, hypermethylation of GSTP1 has been found in urinary sediments after prostate massage in 68% of patients with early confined disease, 78% of patients with locally advanced disease, 29% of patients with PIN and 2% of patients with BPH. These findings resulted in a specificity of 98% and a sensitivity of 73%. The negative predictive value of this test was 80%, which shows that this assay bears great potential to reduce the number of unnecessary biopsies.

Recently, these results were confirmed and a higher frequency of GSTP1 methylation was observed in the urine of men with stage 3 versus stage 2 disease.

Because hypermethylation of GSTP1 has a high specificity for prostate cancer, the presence of GSTP1 hypermethylation in urinary sediments of patients with negative biopsies (33%) and patients with atypia or high-grade PIN (67%) suggests that these patients may have occult prostate cancer.

Recently, a multiplexed assay consisting of 3 methylation markers, *GSTP1, RARB, APC* and an endogenous control was tested on urine samples from patients with serum PSA concentrations ≥2.5 µg/l. A good correlation of *GSTP1* with the number of prostate cancer-positive cores on biopsy was observed. Furthermore, samples that contained methylation for either *GSTP1* or *RARB* correlated with higher tumor volumes. Methylated genes have the potential to provide a new generation of cancer biomarkers.

Micro-array studies have been very useful and informative to identify genes that are consistently up-regulated or down-regulated in prostate cancer compared with benign prostate tissue. These genes can provide prostate cancer-specific biomarkers and give us more insight into the etiology of the disease.

For the molecular diagnosis of prostate cancer, genes that are highly up-regulated in prostate cancer compared to low or normal expression in normal prostate tissue are of special interest. Such genes could enable the detection of one tumor cell in a huge background of normal cells, and could thus be applied as a diagnostic marker in prostate cancer detection.

Differential gene expression analysis has been successfully used to identify prostate cancer-specific biomarkers by comparing malignant with non-malignant prostate tissues. Recently, a new biostatistical method called cancer outlier profile analysis (COPA) was used to identify genes that are differentially expressed in a subset of prostate cancers. COPA identified strong outlier profiles for v-ets erythroblastosis virus E26 oncogene (*ERG*) and ets variant gene 1 (*ETV1*) in 57% of prostate cancer cases. This was in concordance with the results of a study where prostate cancer-associated *ERG* overexpression was found in 72% of prostate cancer cases. In >90% of the cases that overexpressed either *ERG* or *ETV1* a fusion of the 5' untranslated region of the prostate-specific and androgen-regulated transmembrane-serine protease gene (*TMPRSS2*) with these ETS family members was found. Recently, another fusion between *TMPRSS2* and an ETS family member has been described, the *TMPRSS2-ETV4* fusion, although this fusion is sporadically found in prostate cancers.

Furthermore, a fusion of *TMPRSS2* with *ETV5* was found. Overexpression of *ETV5 in vitro* was shown to induce an invasive transcriptional program. These fusions can explain the aberrant androgen-dependent overexpression of ETS family members in subsets of prostate cancer because *TMPRSS2* is androgen-regulated. The discovery of the *TMPRSS2-ERG* gene fusion and the fact that *ERG* is the most-frequently overexpressed proto-oncogene described in malignant prostate epithelial cells suggests its role in prostate tumorigenesis. Fusions of the 5' untranslated region of the *TMPRSS2* gene with the ETS transcription factors *ERG, ETV1* and *ETV4* have been reported in prostate cancer.

Recently, it was shown that non-invasive detection of *TMPRSS2-ERG* fusion transcripts is feasible in urinary sediments obtained after DRE using an RT-PCR-based research assay. Due to the high specificity of the test (93%), the combination of *TMPRSS2-ERG* fusion transcripts with prostate cancer gene 3 (*PCA3*) improved the sensitivity from 62% (*PCA3* alone) to 73% (combined) without compromising the specificity for detecting prostate cancer.

The gene coding for α-methylacyl-CoA racemase (AMACR) on chromosome 5p13 has been found to be consistently up-regulated in prostate cancer. This enzyme plays a critical role in peroxisomal beta oxidation of branched chain fatty acid molecules obtained from dairy and beef. Interestingly, the consumption of dairy and beef has been associated with an increased risk for prostate cancer.

In clinical prostate cancer tissue, a 9-fold over-expression of AMACR mRNA has been found compared to normal prostate tissue. Immunohistochemical (IHC) studies and Western blot analyses have confirmed the up-regulation of AMACR at the protein level. Furthermore, it has been shown that 88% of prostate cancer cases and both untreated metastases and hormone refractory prostate cancers were strongly positive for AMACR. AMACR expression has not been detected in atrophic glands, basal cell hyperplasia and urothelial epithelium or metaplasia. IHC studies also showed that AMACR expression in needle biopsies had a 97% sensitivity and a 100% specificity for prostate cancer detection.

Combined with a staining for p63, a basal cell marker that is absent in prostate cancer, AMACR greatly facilitated the identification of malignant prostate cells. Its high expression and cancer-cell specificity implicate that AMACR may also be a candidate for the development of molecular probes which may facilitate the identification of prostate cancer using non-invasive imaging modalities.

There have been many efforts to develop a body fluid-based assay for AMACR. A small study indicated that *AMACR*-based quantitative real-time PCR analysis on urine samples obtained after prostate massage has the potential to exclude the patients with clinically insignificant disease when *AMACR* mRNA expression is normalized for PSA. Western blot analysis on urine samples obtained after prostate massage had a sensitivity of 100%, a specificity of 58%, a positive predictive value (PPV) of 72%, and a negative predictive value (NPV) of 88% for prostate cancer. These assays using *AMACR* mRNA for the detection of prostate cancer in urine specimens are promising.

Using cDNA micro-array analysis, it has been shown that hepsin, a type II transmembrane serine protease, is one of the most-differentially over-expressed genes in prostate cancer compared to normal prostate tissue and BPH tissue. Using a quantitative real-time PCR analysis it has been shown that hepsin is over-expressed in 90% of prostate cancer tissues. In 59% of the prostate cancers this over-expression was more than 10-fold.

Also there has been a significant correlation between the up-regulation of hepsin and tumor-grade. Further studies will have to determine the tissue-specificity of hepsin and the diagnostic value of this serine protease as a new serum marker. Since hepsin is up-regulated in advanced and more aggressive tumors it suggests a role as a prognostic tissue marker to determine the aggressiveness of a tumor.

Telomerase, a ribonucleoprotein, is involved in the synthesis and repair of telomeres that cap and protect the ends of eukaryotic chromosomes. The human telomeres consist of tandem repeats of the TTAGGG sequence as well as several different binding proteins. During cell division telomeres cannot be fully replicated and will become shorter. Telomerase can lengthen the telomeres and thus prevents the shortening of these structures. Cell division in the absence of telomerase activity will lead to shortening of the telomeres. As a result, the lifespan of the cells becomes limited and this will lead to senescence and cell death.

In tumor cells, including prostate cancer cells, telomeres are significantly shorter than in normal cells. In cancer cells with short telomeres, telomerase activity is required to escape senescence and to allow immortal growth. High telomerase activity has been found in 90% of prostate cancers and was shown to be absent in normal prostate tissue.

In a small study on 36 specimens telomerase activity has been used to detect prostate cancer cells in voided urine or urethral washing after prostate massage. This test had a sensitivity of 58% and a specificity of 100%. The negative predictive value of the test was 55%.

Although it has been a small and preliminary study, the low negative predictive value indicates that telomerase activity measured in urine samples is not very promising in reducing the number of unnecessary biopsies.

The quantification of the catalytic subunit of telomerase, hTERT, showed a median over-expression of hTERT mRNA of 6-fold in prostate cancer tissues compared to normal prostate tissues. A significant relationship was found between hTERT expression and tumor stage, but not with Gleason score. The quantification of hTERT using real-time PCR showed that hTERT could well discriminate prostate cancer tissues from non-malignant prostate tissues. However, hTERT mRNA is expressed in leukocytes, which are regularly present in body fluids such as blood and urine. This may cause false positivity. As such, quantitative measurement of hTERT in body fluids is not very promising as a diagnostic tool for prostate cancer.

Prostate-specific membrane antigen (PSMA) is a transmembrane glycoprotein that is expressed on the surface of prostate epithelial cells. The expression of PSMA appears to be restricted to the prostate. It has been shown that PSMA is upregulated in prostate cancer tissue compared with benign prostate tissues. No overlap in PSMA expression has been found between BPH and prostate cancer, indicating that PSMA is a very promising diagnostic marker.

Recently, it has been shown that high PSMA expression in prostate cancer cases correlated with tumor grade, pathological stage, aneuploidy and biochemical recurrence. Furthermore, increased *PSMA* mRNA expression in primary prostate cancers and metastasis correlated with PSMA protein overexpression. Its clinical utility as a diagnostic or prognostic marker for prostate cancer has been hindered by the lack of a sensitive immunoassay for this protein. However, a combination of ProteinChip® (Ciphergen Biosystems) arrays and SELDI-TOF MS has led to the introduction of a protein biochip immunoassay for the quantification of serum PSMA. It was shown that the average serum PSMA levels for prostate cancer patients were significantly higher compared with those of men with BPH and healthy controls. These findings implicate a role for serum PSMA to distinguish men with BPH from prostate cancer patients. However, further studies are needed to assess its diagnostic value.

A combination of ProteinChip® arrays and SELDI-TOF MS has led to the introduction of a protein biochip immunoassay for the quantification of serum PSMA. It was shown that the average serum PSMA levels for prostate cancer patients were significantly higher compared with those of men with BPH and healthy controls. These findings implicate a role for serum PSMA to distinguish men with BPH from prostate cancer patients. However, further studies are needed to assess its diagnostic value.

RT-PCR studies have shown that PSMA in combination with its splice variant PSM' could be used as a prognostic marker for prostate cancer. In the normal prostate, PSM' expression is higher than PSMA expression. In prostate cancer tissues, the PSMA expression is more dominant. Therefore, the ratio of PSMA to PSM' is highly indicative for disease progression. Designing a quantitative PCR analysis which discriminates between the two PSMA forms could yield another application for PSMA in diagnosis and prognosis of prostate cancer.

Because of its specific expression on prostate epithelial cells and its upregulation in prostate cancer, PSMA has become the target for therapies. The proposed strategies range from targeted toxins and radio nuclides to immunotherapeutic agents. First-generation products have entered clinical testing.

Delta-catenin (p120/CAS), an adhesive junction-associated protein, has been shown to be highly discriminative between BPH and prostate cancer. *In situ* hybridization studies showed the highest expression of δ-catenin transcripts in adenocarcinoma of the prostate and low to no expression in BPH tissue. The average over-expression of δ-catenin in prostate cancer compared to BPH is 15.7 fold.

Both quantitative PCR and in situ hybridization analysis could not find a correlation between δ-catenin expression and Gleason scores.

Increased δ-catenin expression in human prostate cancer results in alterations of cell cycle and survival genes, thereby promoting tumor progression. δ-catenin was detected in cell-free human voided urine prostasomes. The δ-catenin immunoreactivity was significantly increased in the urine of prostate cancer patients. Further studies are needed to assess its potential utility in the diagnosis of prostate cancer.

PCA3, formerly known as DD3, has been identified using differential display analysis. PCA3 was found to be highly over-expressed in prostate tumors compared to normal prostate tissue of the same patient using Northern blot analysis. Moreover, PCA3 was found to be strongly over-expressed in more than 95% of primary prostate cancer specimens and in prostate cancer metastasis. Furthermore, the expression of PCA3 is restricted to prostatic tissue, i.e. no expression has been found in other normal human tissues.

The gene encoding for PCA3 is located on chromosome 9q21.2. The PCA3 mRNA contains a high density of stop-codons. Therefore, it lacks an open reading frame resulting in a non-coding RNA. Recently, a time-resolved quantitative RT-PCR assay (using an internal standard and an external calibration curve) has been developed. The accurate quantification power of this assay showed a median 66-fold up-regulation of PCA3 in prostate cancer tissue compared to normal prostate tissue. Moreover, a median-up-regulation of 11-fold was found in prostate tissues containing less than 10% of prostate cancer cells. This indicated that PCA3 was capable to detect a small number of tumor cells in a huge background of normal cells.

This hypothesis has been tested using the quantitative RT-PCR analysis on voided urine samples. These urine samples were obtained after digital rectal examination (DRE) from a group of 108 men who were indicated for prostate biopsies based on a total serum PSA value of more than 3 ng/ml. This test had 67% sensitivity and 83% specificity using prostatic biopsies as a gold-standard for the presence of a tumor. Furthermore, this test had a negative predictive value of 90%, which indicates that the quantitative determination of PCA3 transcripts in urinary sediments obtained after extensive prostate massage bears great potential in the reduction of the number of invasive TRUS guided biopsies in this population of men.

The tissue-specificity and the high over-expression in prostate tumors indicate that PCA3 is the most prostate cancer-specific gene described so far. Gen-probe Inc. has the exclusive worldwide licence to the PCA3 technology. Multicenter studies using the validated PCA3 assay can provide the first basis for the molecular diagnostics in clinical urological practice.

Modulated expression of cytoplasmic proteins HSP-27 and members of the PKC isoenzyme family have been correlated with prostate cancer progression.

Modulation of expression has clearly identified those cancers that are aggressive - and hence those that may require urgent treatment, irrespective of their morphology. Although not widely employed, antibodies to these proteins are authenticated, are available commercially and are straightforward in their application and interpretation, particularly in conjunction with other reagents as double-stained preparations.

The significance of this group of markers is that they accurately distinguish prostate cancers of aggressive phenotype. Modulated in their expression by invasive cancers, when compared to non-neoplastic prostatic tissues, those malignancies which express either HSP27 or PKCβ at high level invariably exhibit a poor clinical outcome. The mechanism of this association warrants elucidation and validation.

E2F transcription factors, including E2F3 located on chromosome 6p22, directly modulate expression of EZH2. Overexpression of the EZH2 gene has been important in development of human prostate cancer.

Varambally and collegues identified EZH2 as a gene overexpressed in hormone-refractory metastatic prostate cancer and showed that patients with clinically localized prostate cancers that express EZH2 have a worse progression than those who do not express the protein.

Using tissue microarrays, expression of high levels of nuclear E2F3 occurs in a high proportion of human prostate cancers but is a rare event in non-neoplastic prostatic epithelium. These data, together with other published information, suggested that the pRB-E2F3-EZH2 control axis may have a crucial role in modulating aggressiveness of individual human prostate cancers.

The prime challenge for molecular diagnostics is the identification of clinically insignificant prostate cancer, i.e. separate the biologically aggressive cancers from the indolent tumors. Furthermore, markers predicting and monitoring the response to treatment are urgently needed.

In current clinical settings over diagnosis and over treatment become more and more manifest, further underlining the need for biomarkers that can aid in the accurate identification of the patients that do not- and do-need treatment.

The use of AMACR immunohistochemistry is now used in the identification of malignant processes in the prostate thus aiding the diagnosis of prostate cancer. Unfortunately, the introduction of molecular markers on tissue as prognostic tool has not been validated for any of the markers discussed.

Experiences over the last two decades have revealed the practical and logistic complexity in translating molecular markers into clinical use. Several prospective efforts, taking into account these issues, are currently ongoing to establish clinical utility of a number of markers. Clearly, tissue biorepositories of well documented specimens, including clinical follow up data, play a pivotal role in the validation process.

Novel body fluid tests based on GSTP1 hypermethylation and the gene PCA3, which is highly over-expressed in prostate cancer, enabled the detection of prostate cancer in non-invasively obtained body fluids such as urine or ejaculates.

The application of new technologies has shown that a large number of genes are up-regulated in prostate cancer. For example, WO 2009/042742 discloses a method for in vitro diagnosing prostate cancer in a human individual by determining DLX1 expression, establishing up or down regulation thereof and subsequently diagnosing prostate cancer.

Although the makers outlined above, at least partially, address the need in the art for tumor markers, and especially prostate tumor markers, there is a continuing need for reliable (prostate) tumor markers, and especially markers indicative of the course of the disease.

It is an object of the present invention, amongst others, to meet at least partially, if not completely, the above object.

According to the present invention, the above object, amongst others, is met by tumor markers and methods as outlined in the appended claims.

Specifically, the above object, amongst others, is met by a method for in vitro diagnosing prostate cancer in a human individual comprising:
- determining the expression of DLX1; and
- establishing up or down regulation of expression of DLX1 as compared to expression of DLX1 in a sample from an individual without prostate cancer;
thereby providing said diagnosis of prostate cancer.

According to the present invention diagnosing prostate cancer preferably comprises diagnosis, prognosis and/or prediction of disease survival.

According to the present invention, expression analysis comprises establishing an increased or decreased expression of a gene as compared to expression of the gene in a non-prostate cancer tissue, i.e., under non-disease conditions.

According to a preferred embodiment, the present method is performed on urinary, preferably urinary sediment samples.

According to a preferred embodiment of the present method, determining the expression comprises determining mRNA expression of said one or more genes.

Expression analysis based on mRNA is generally known in the art and routinely practiced in diagnostic labs world-wide. For example, suitable techniques for mRNA analysis are Northern blot hybridisation and amplification based techniques such as PCR, and especially real time PCR, and NASBA.

According to a particularly preferred embodiment, expression analysis comprises high-throughput DNA array chip analysis not only allowing the simultaneous analysis of multiple samples but also an automatic analysis processing.

The present method provides diagnosing of prostate cancer in a human individual selected from the group consisting of diagnosing high grade PrCa (HG), PrCa Met and CRPC.

LG indicates low grade PrCa (Gleason Score equal or less than 6) and represent patients with good prognosis. HG indicates high grade PrCa (Gleason Score of 7 or more) and represents patients with poor prognosis. CRPC indicates castration resistant prostate cancer and represents patients with aggressive localized disease. Finally, PrCa Met represents patients with poor prognosis.

According to a particularly preferred embodiment of the present method, the present invention provides diagnosis of CRPC.

Considering the diagnosing value of the present genes as biomarkers for prostate cancer, the present invention also relates to the use of DLX1 for in vitro diagnosing the present prostate cancer.

In the present description, reference is made to genes suitable as biomarkers for prostate cancer by referring to their arbitrarily assigned names. Although the skilled person is readily capable to identify and use the present genes as biomarkers based on these names, the appended figures provide both the cDNA sequence and protein sequences of these genes in the public database as also the references disclosing these genes. Based on the data provided in the figures, the skilled person, without undue experimentation and using standard molecular biology means, will be capable of determining the expression of the indicated biomarkers in a sample thereby providing the present method of diagnosis.

The present invention will be further elucidated in the following examples. In the examples, reference is made to figures, wherein:

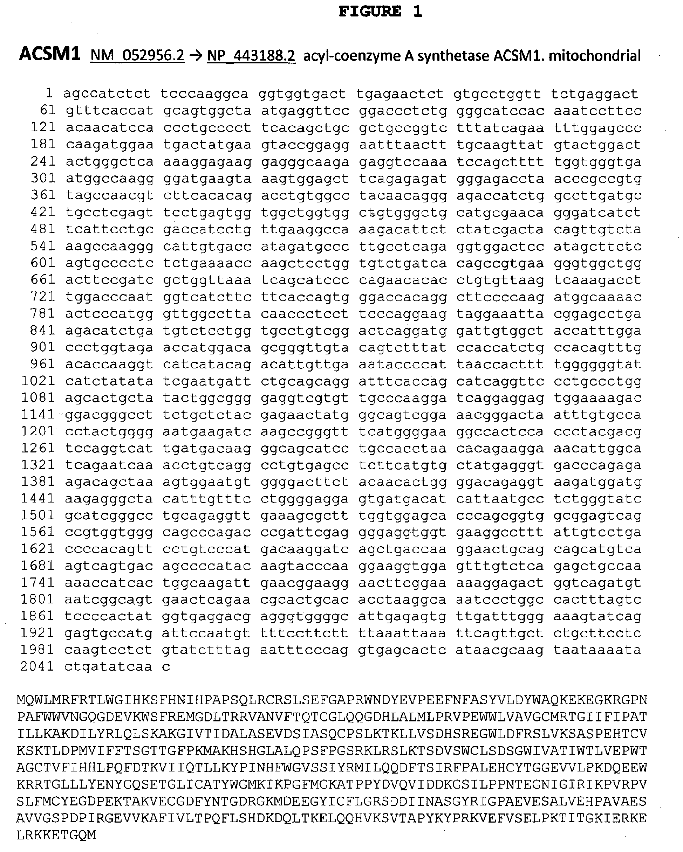

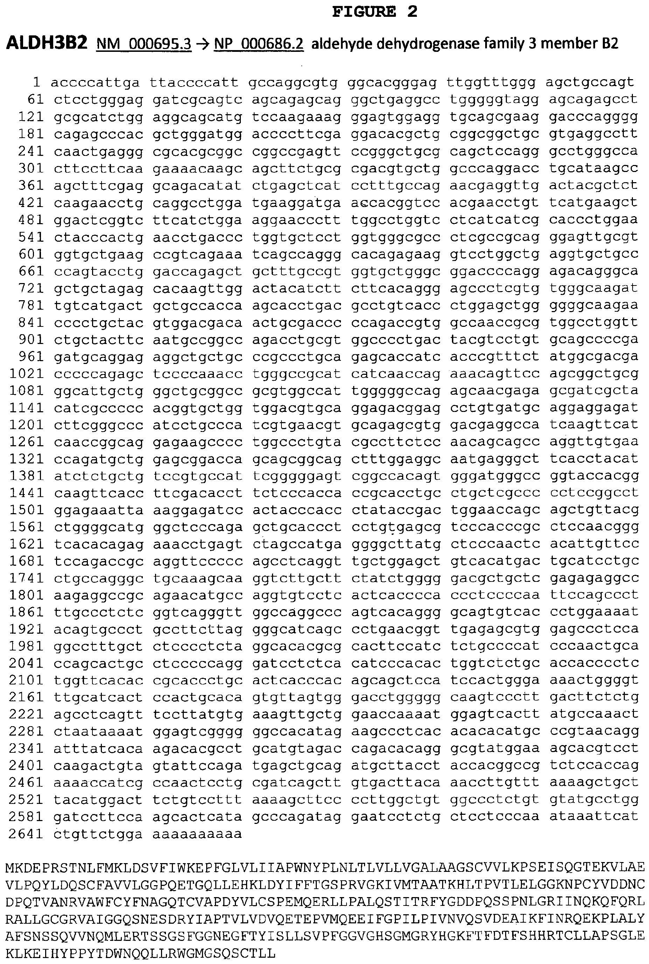

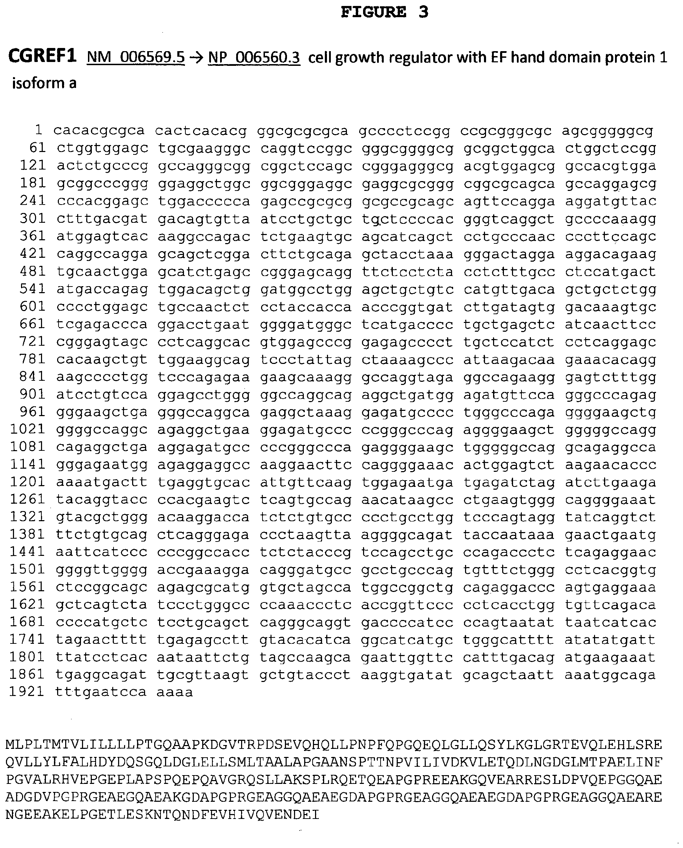

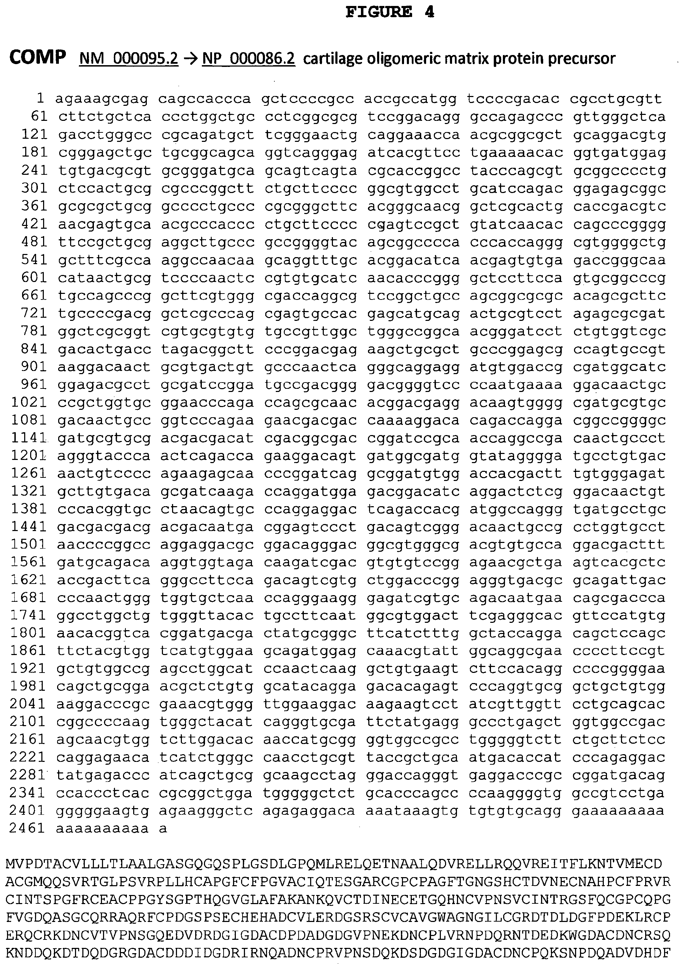

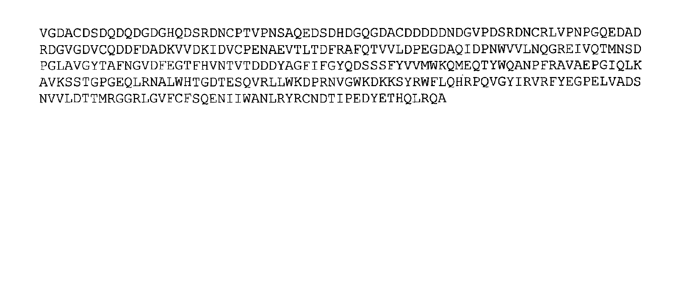

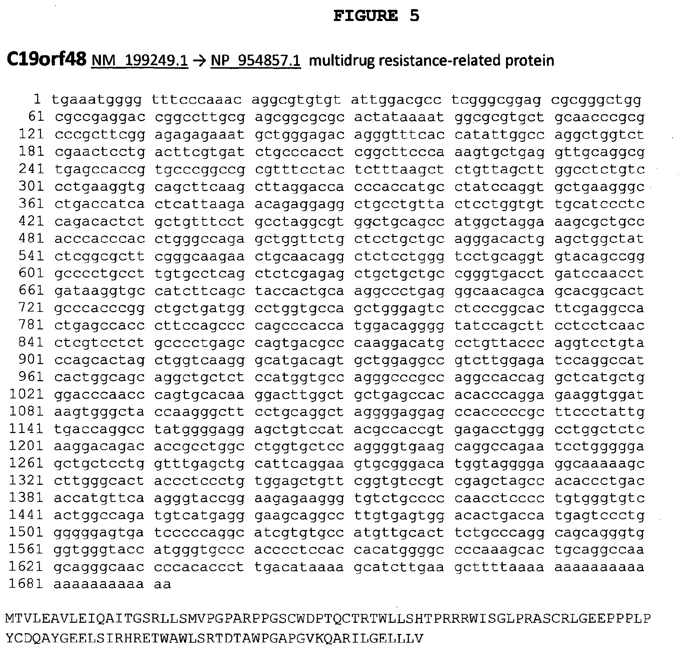

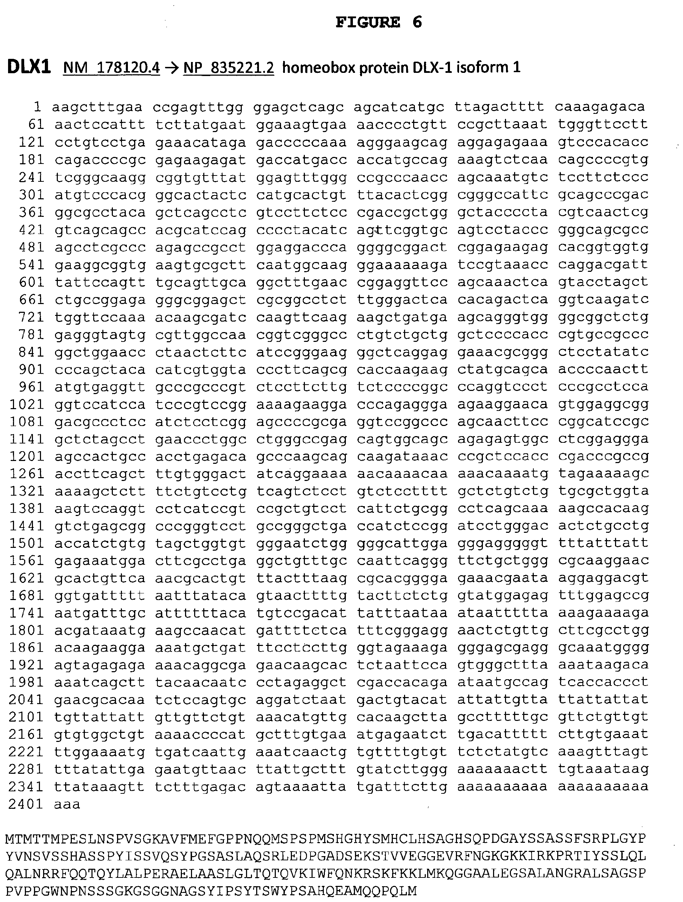

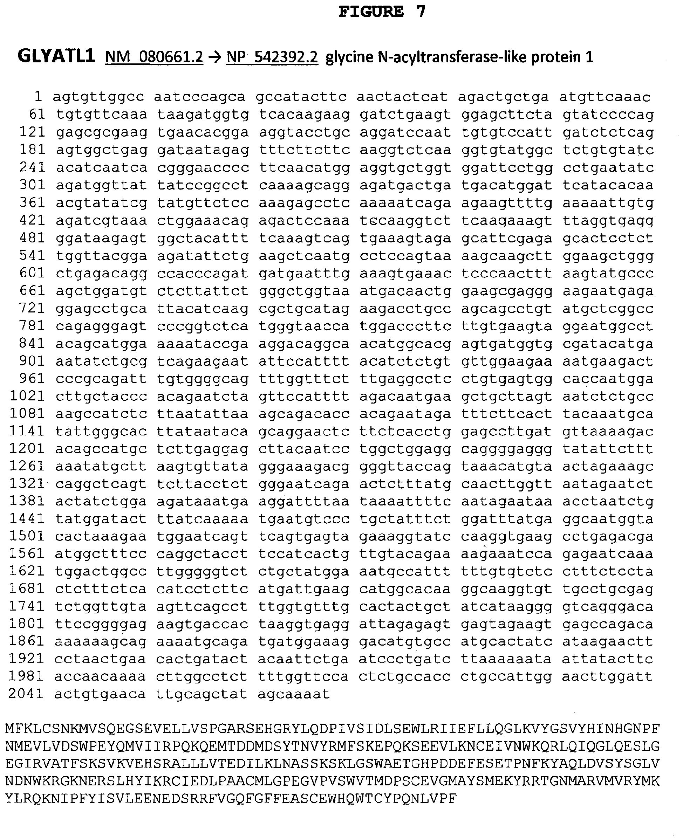

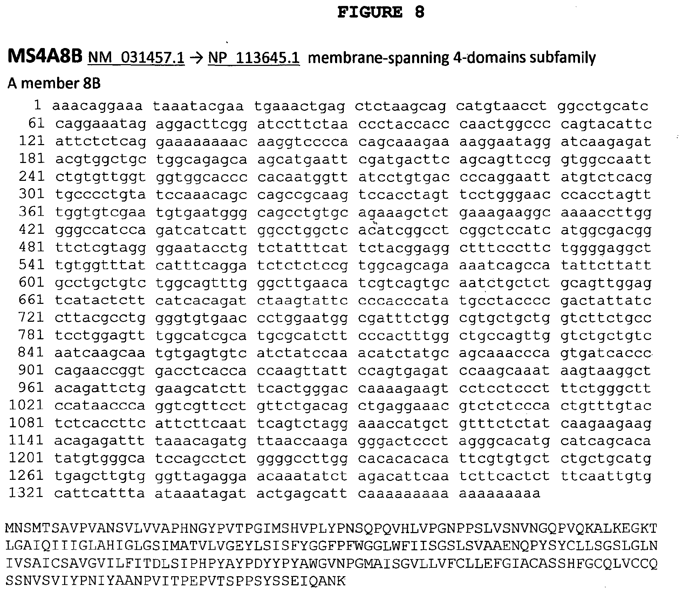

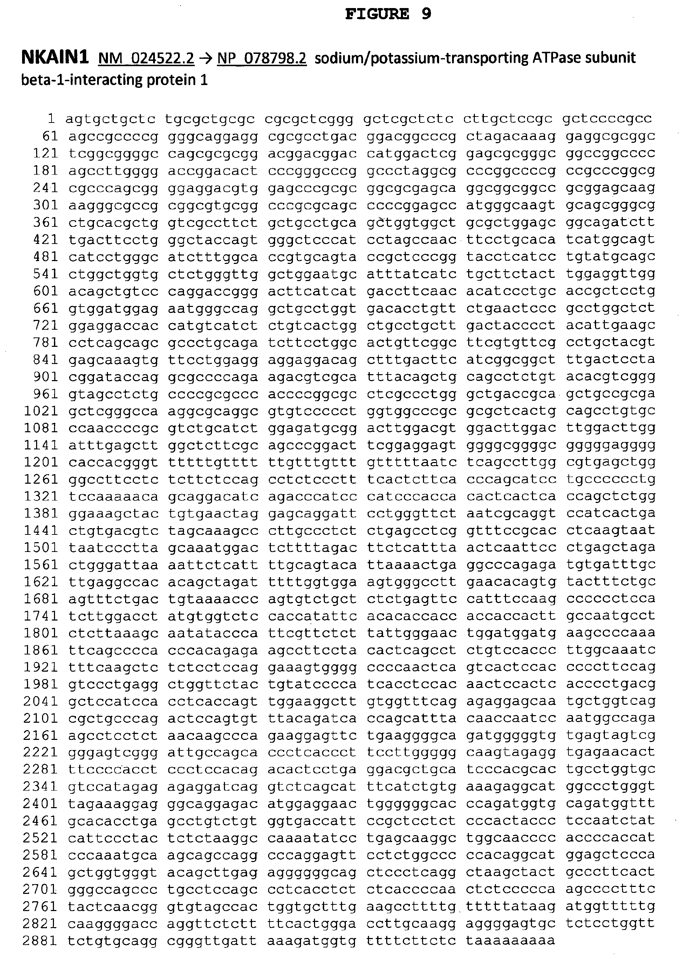

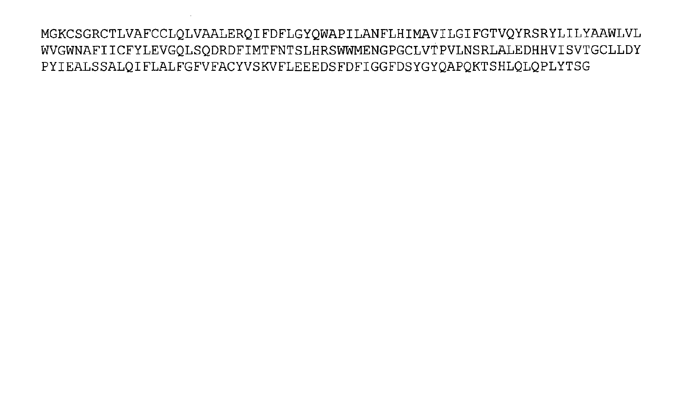

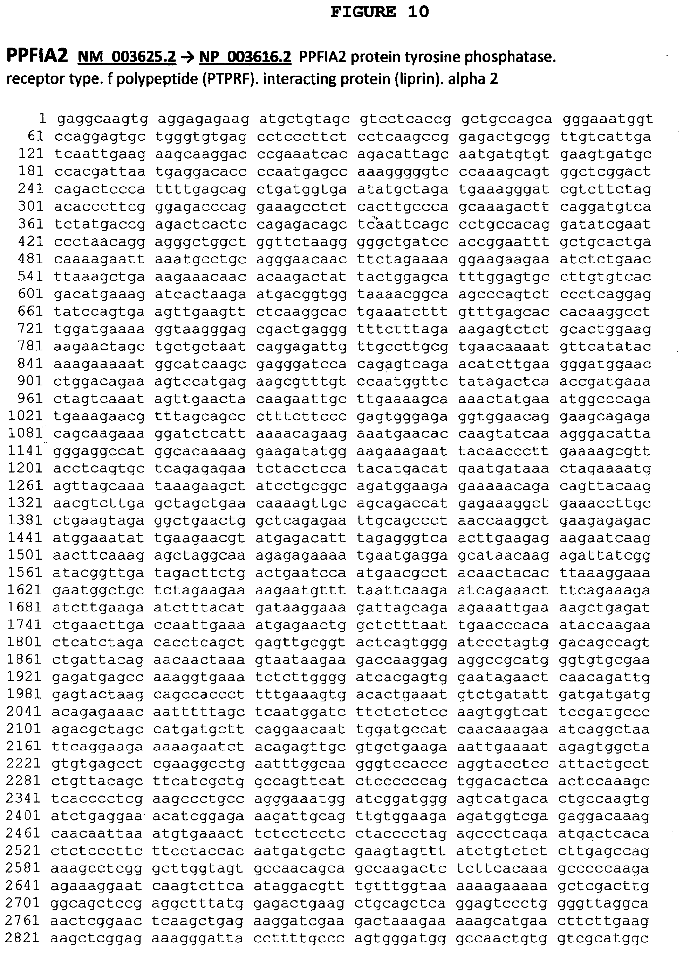

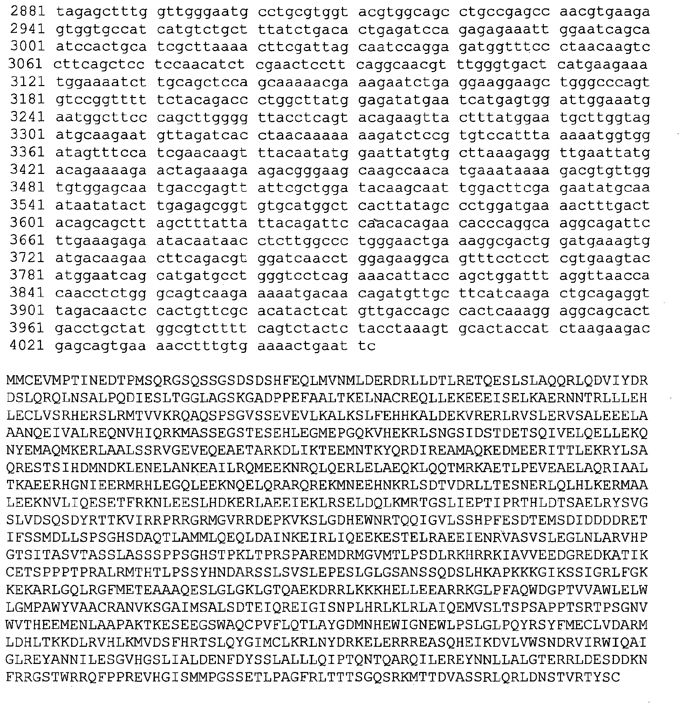

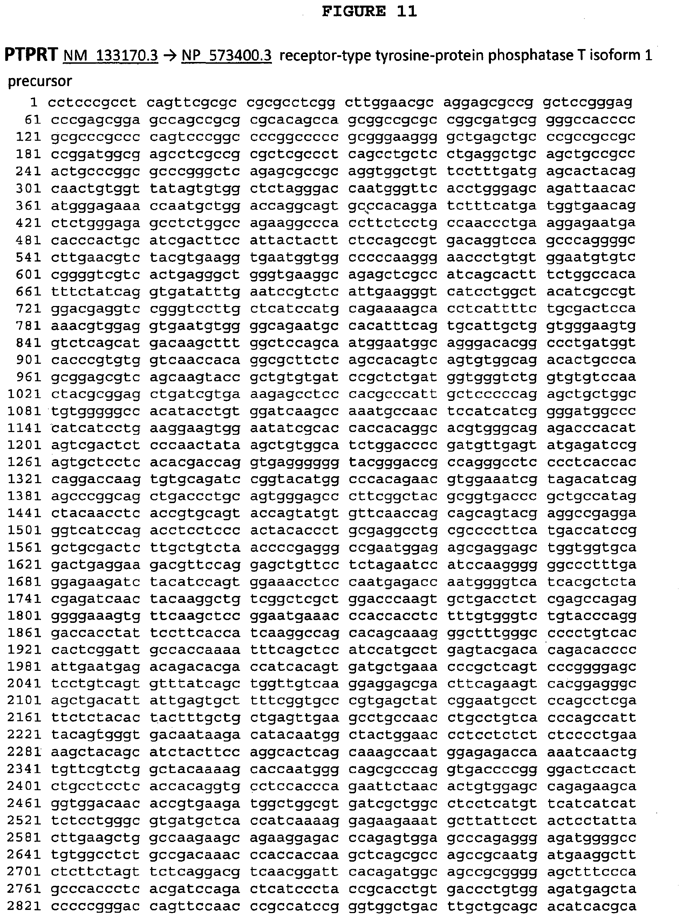

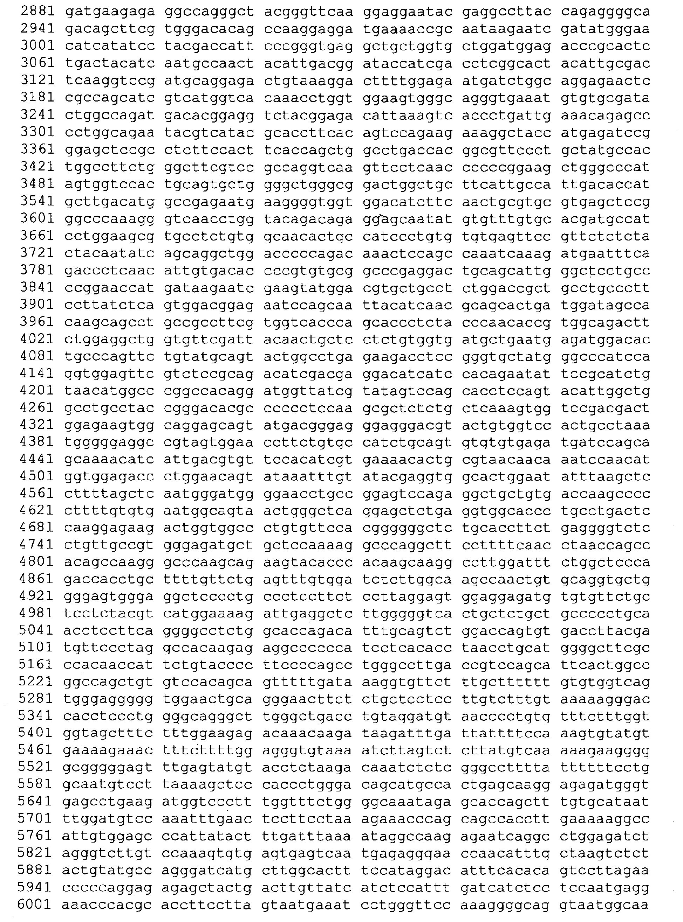

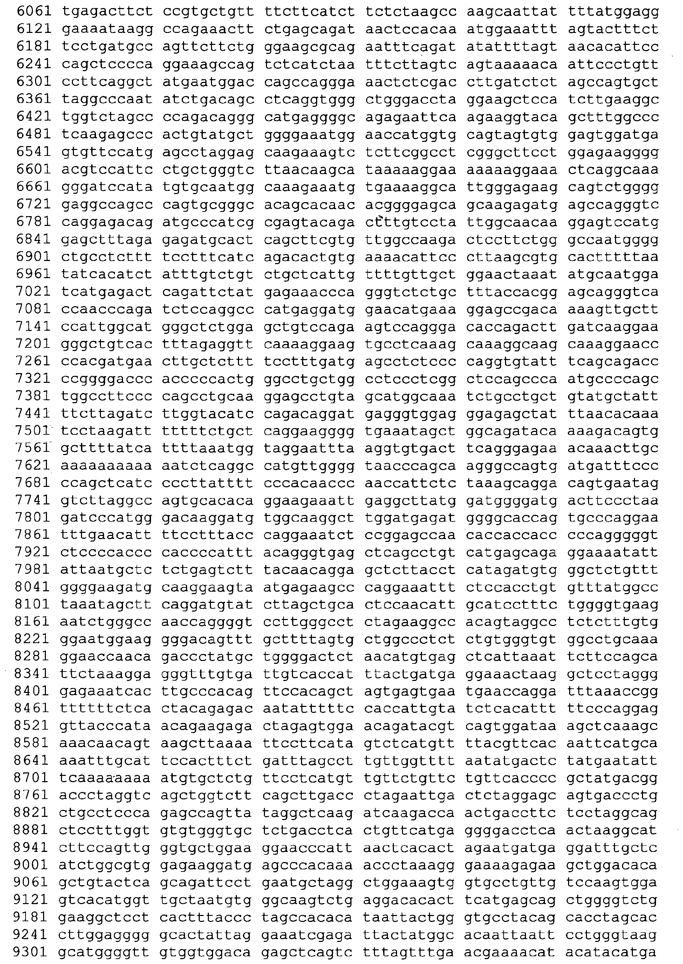

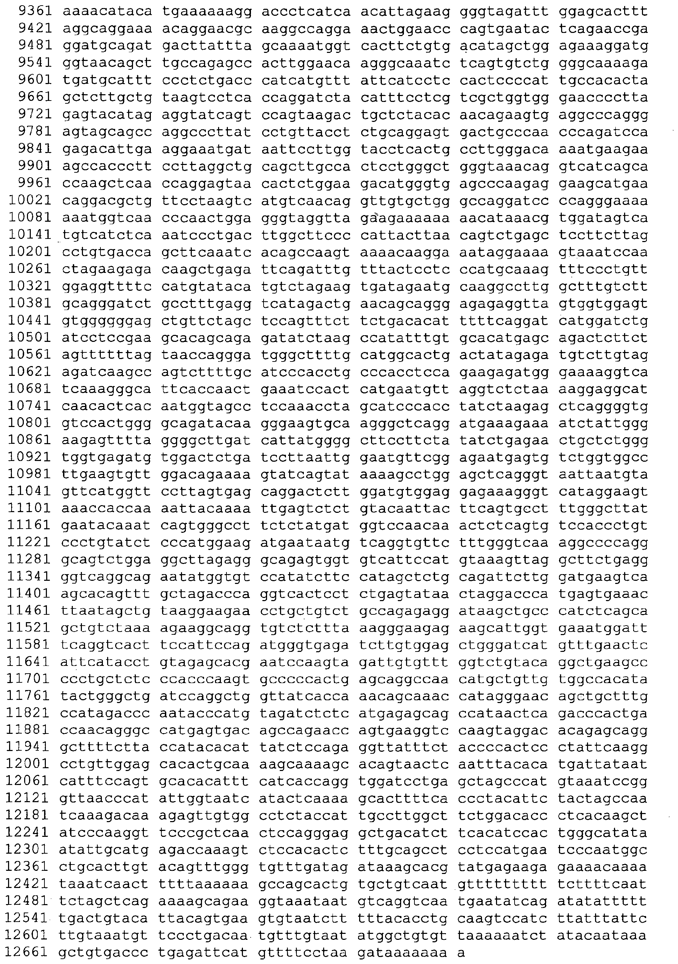

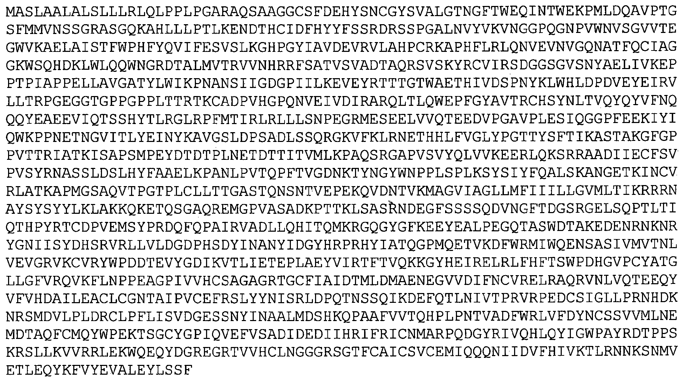

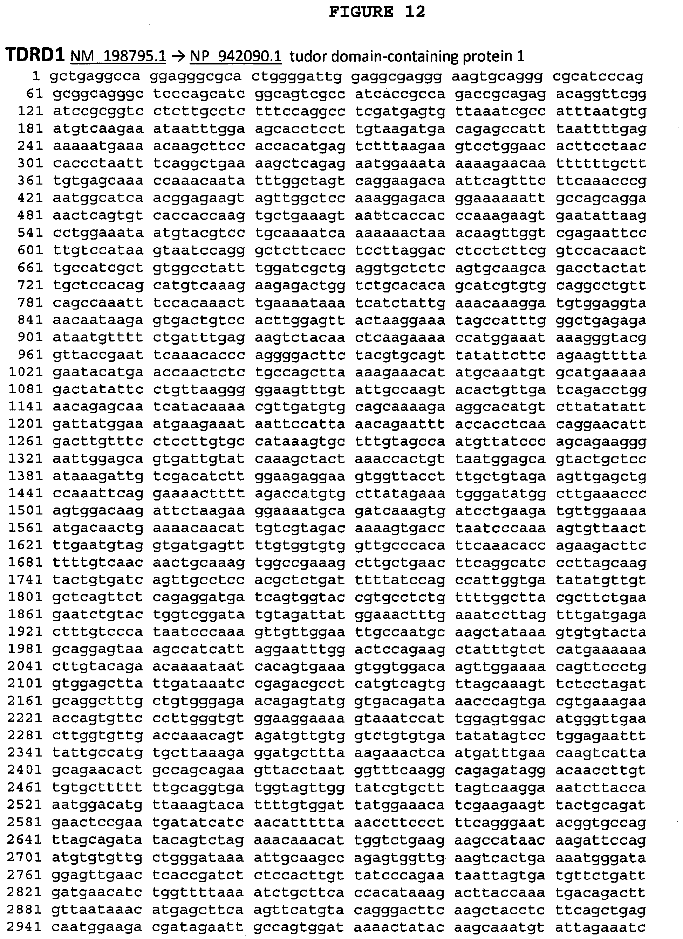

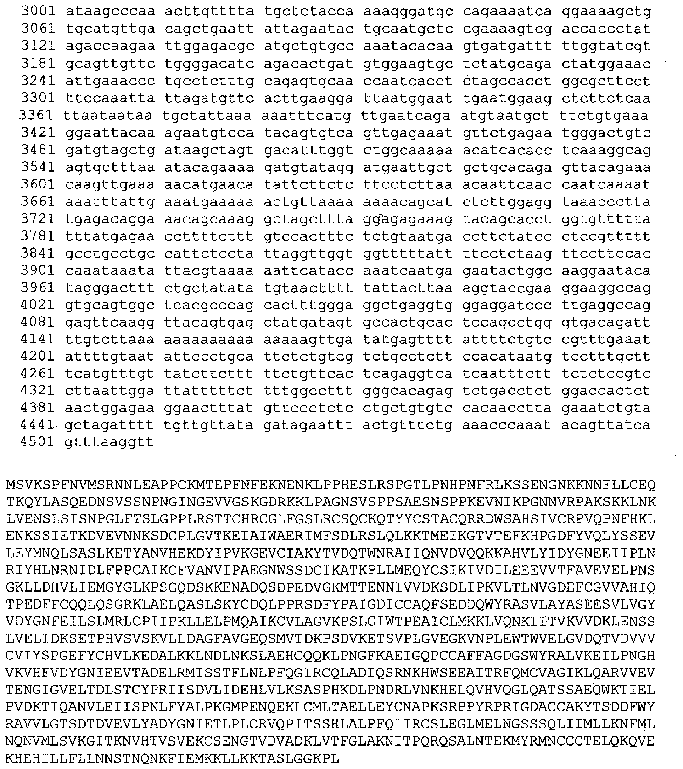

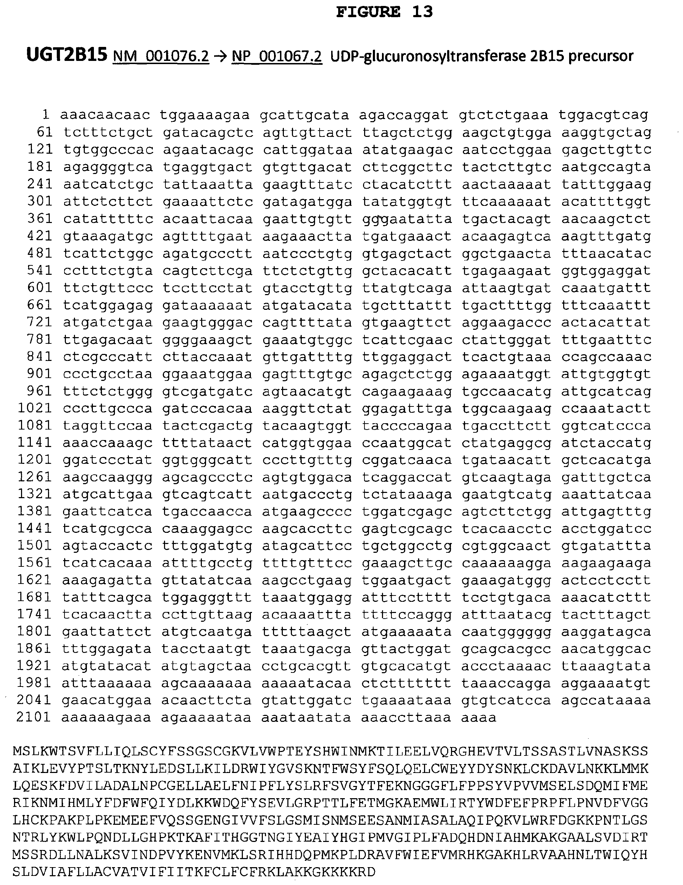

- **Figures 1-13:**: show the mRNA and amino acid sequences of the ACSM1 gene (NM_052956, NP_443188); the ALDH3B2 gene (NM_000695, NP_000686); the CGREF1 gene (NM_006569, NP_006560);the COMP gene (NM_000095, NP_000086): the C19orf48 gene (NM_199249, NP_954857); the DLX1 gene (NM_178120, NP_835221); the GLYATL1 gene (NM_080661, NP_542392); the MS4A8B gene (NM_031457, NP_113645); the NKAIN1 gene (NM_024522, NP_078798); the PPFIA2 gene (NM_003625, NP_003616); the PTPRT gene (NM_133170, NP_573400); the TDRD1 gene (NM_198795, NP_942090); and the UGT2B15 gene (NM_001076, NP_001067).

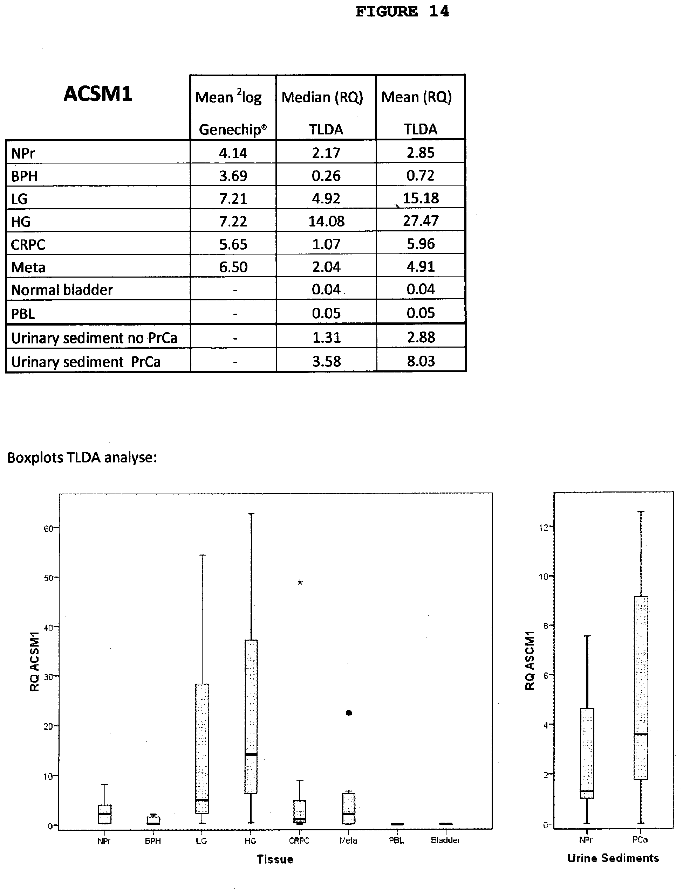

- **Figures 14-26:**: show boxplots based on the TLDA validation data on the groups normal prostate (NPr), BPH, low grade prostate cancer (LG PrCa), HG, high grade prostate cancer (HG PrCa), CRPC, prostate cancer metastasis (PrCa Met), normal bladder, peripheral blood lymphocytes (PBL) and urinary sediments.

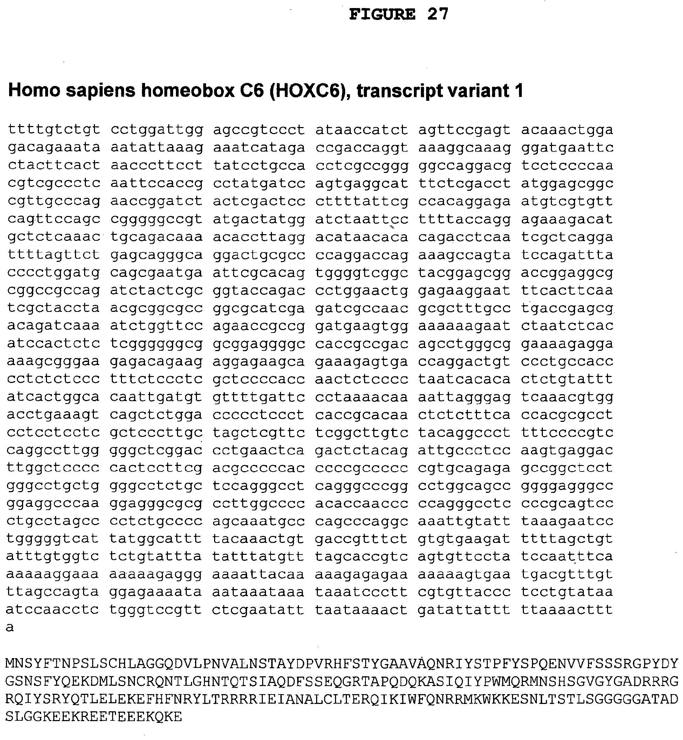

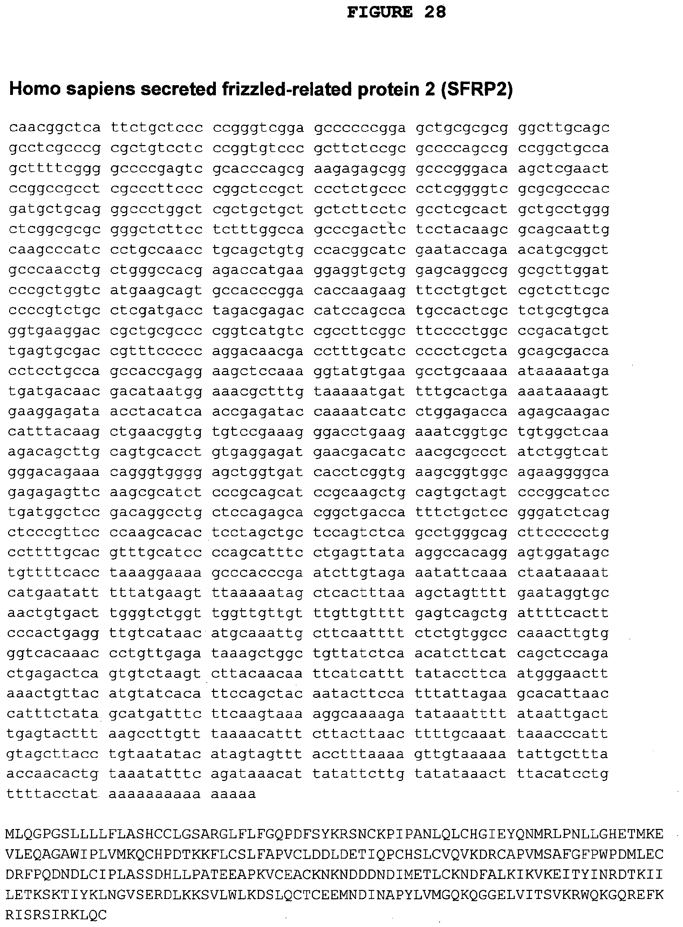

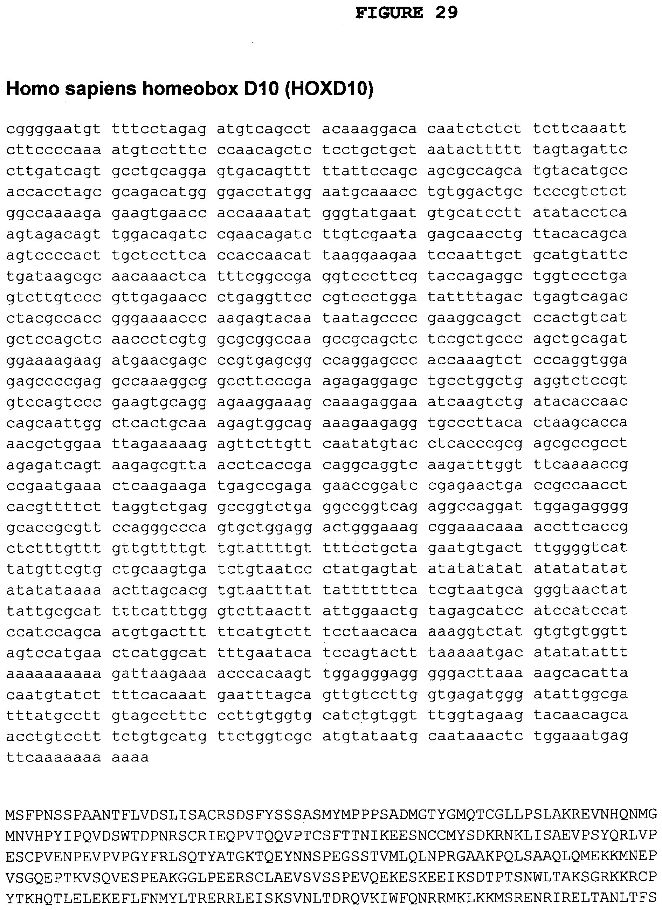

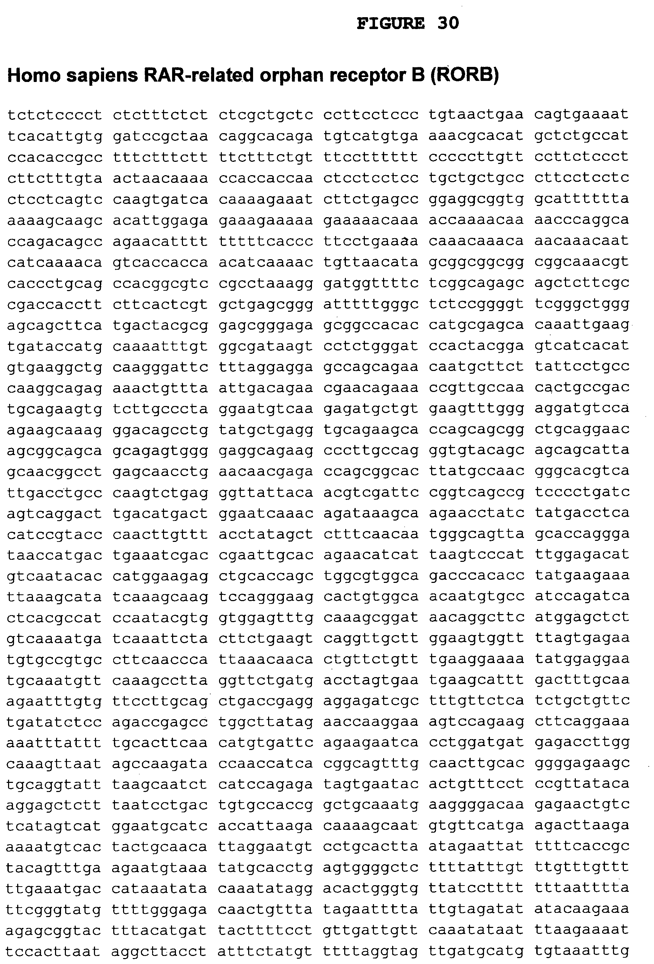

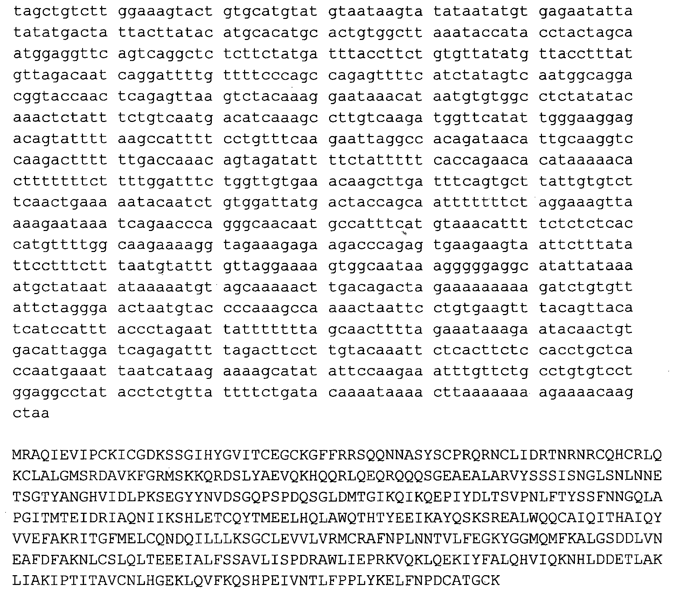

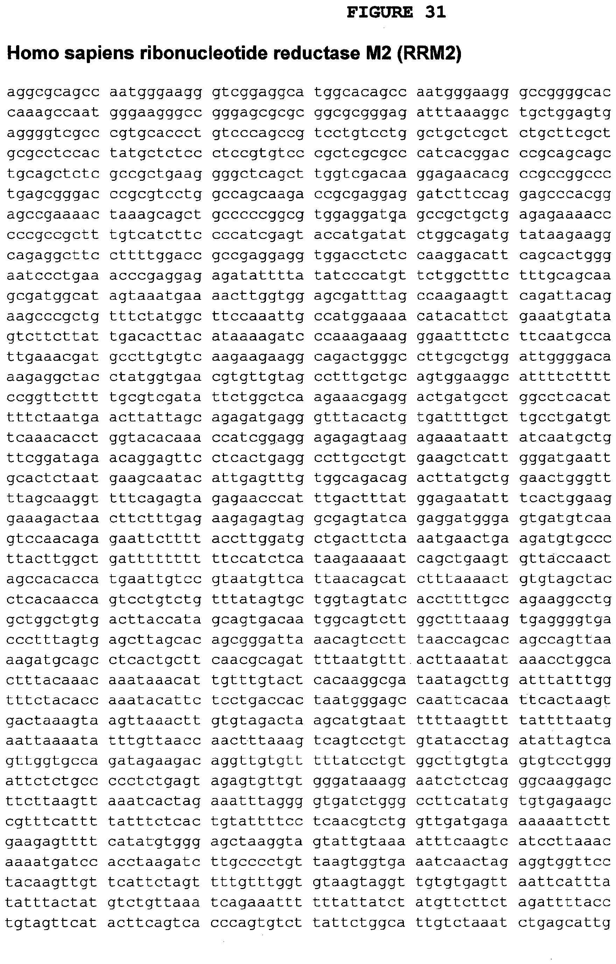

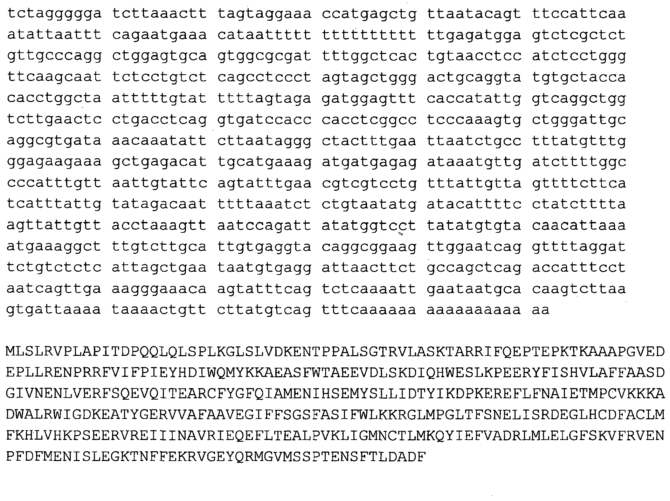

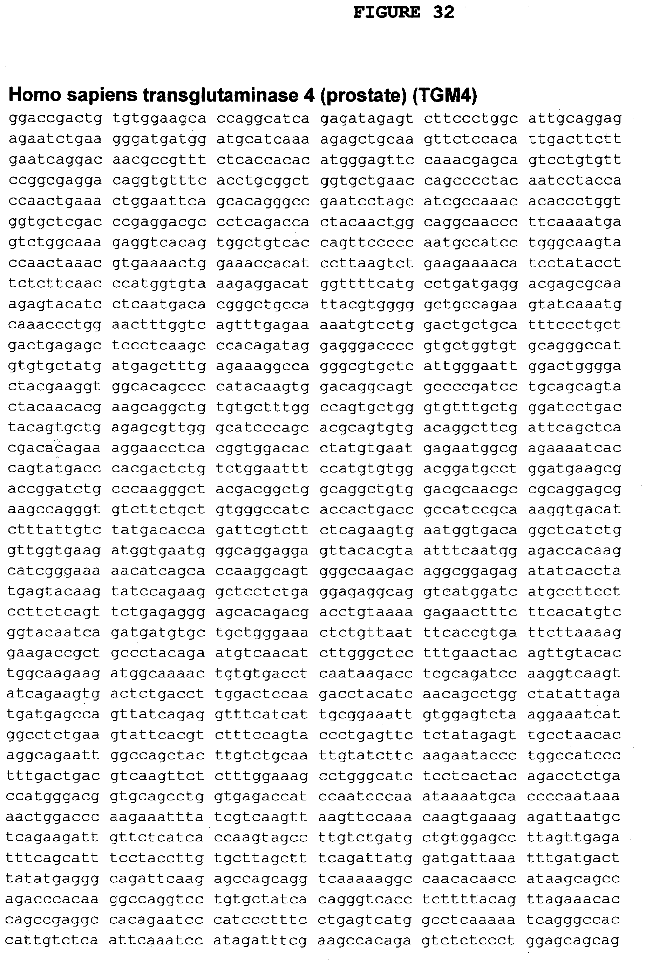

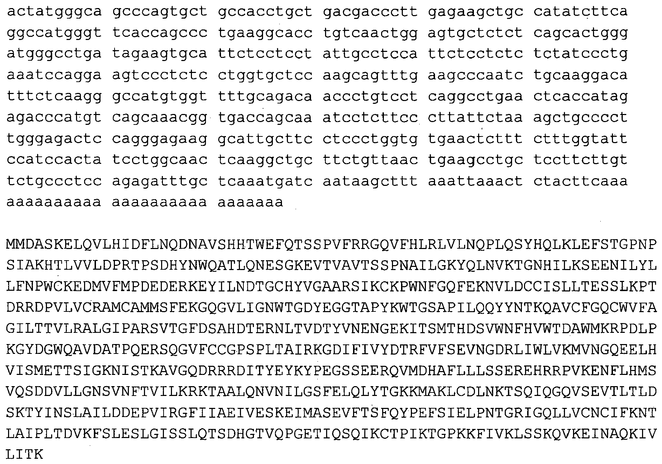

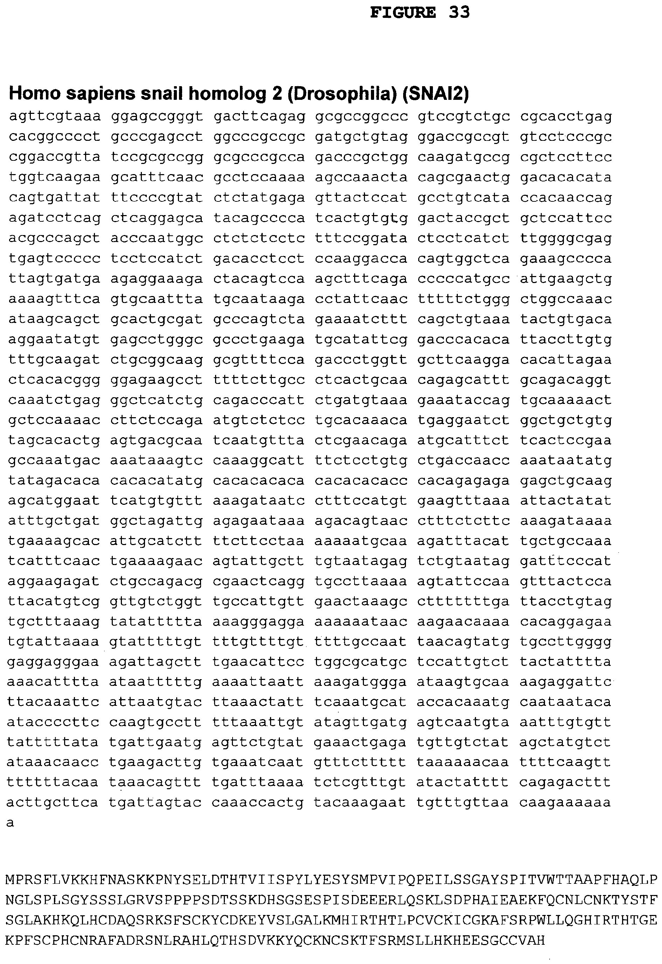

- **Figures 27-33:**: show the cDNA and amino acid sequences of the HOXC6 gene (NM_004503.3, NP_004494.1); the SFRP2 gene (NM_003013.2, NP_003004.1); the HOXD10 gene (NM_002148.3, NP_002139.2); the RORB gene (NM_006914.3, NP_008845.2); the RRM2 gene (NM_001034.2, NP_001025.1); the TGM4 gene (NM_003241.3, NP_003232.2); and the SNAI2 gene (NM_003068.3, NP_003059.1, respectively;

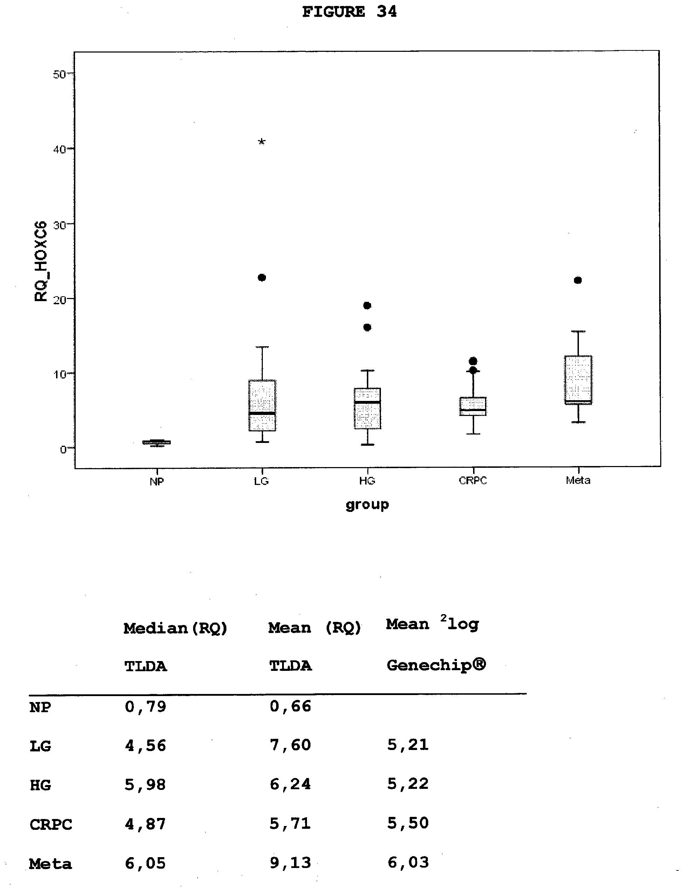

- **Figures 34-40:**: show boxplot TLDA data based on group LG (low grade), HG (high grade), CRPC (castration resistant) and PrCa Met (prostate cancer metastasis) expression analysis of HOXC6 gene (NM_004503.3); the SFRP2 gene (NM_003013.2); the HOXD10 gene (NM_002148.3); the RORB gene (NM_006914.3,); the RRM2 gene (NM_001034.2); the TGM4 gene (NM_003241.3); and the SNAI2 gene (NM_003068.3), respectively. NP indicates no prostate cancer, i.e., normal or standard expression levels.

### Example 1

To identify markers for aggressive prostate cancer, the gene expression profile (Affymetrix exon 1.0 arrays) of samples from patients with prostate cancer in the following categories were used:
Prostate samples in the following categories were used:
   - Normal prostate (NPr), n=8.
   - Benign Prostatic Hyperplasia (BPH), n=12.
   - Low grade prostate cancer (LG PrCa): tissue specimens from primary tumors with a Gleason Score ≤6 obtained after radical prostatectomy. This group represents patients with a good prognosis, n=25.

   - High grade prostate cancer (HG PrCa): tissue specimens from primary tumors with a Gleason Score ≥7 obtained after radical prostatectomy. This group represents patients with poor prognosis, n=24.
   - Castration resistant prostate cancer (CRPC): tissue specimens are obtained from patients that are progressive under endocrine therapy and who underwent a transurethral resection of the prostate (TURP),n=23
   - Prostate cancer metastases (PrCa Met): tissue specimens are obtained from positive lymfnodes after LND or after autopsy. This group represents patients with poor prognosis, n=7.
Furthermore, for diagnosing clinically significant prostate cancer (patients with a poor prognosis), the expression profiles of the categories pT2 (tumor confined to the prostate, n=10) and pT3 (locally advanced prostate cancer, n=9) were determined.

The expression analysis is performed according to standard protocols.

Briefly, from patients with prostate cancer (belonging to one of the last four previously mentioned categories) tissue was obtained after radical prostatectomy or TURP. Normal prostate was obtained from cancer free regions of these samples or from autopsy. BPH tissue was obtained from TURP or transvesical open prostatectomy (Hryntschak). The tissues were snap frozen and cryostat sections were H.E. stained for classification by a pathologist.

Tumor- and tumor free areas were dissected and total RNA was extracted with TRIzol (Invitrogen, Carlsbad, CA, USA) following manufacturer's instructions. The total RNA was purified with the Qiagen RNeasy mini kit (Qiagen, Valencia, CA, USA). Integrity of the RNA was checked by electrophoresis using the Agilent 2100 Bioanalyzer.

From the purified total RNA, 1 µg was used for the GeneChip Whole Transcript (WT) Sense Target Labeling Assay. (Affymetrix, Santa Clara, CA, USA). According to the protocol of this assay, the majority of ribosomal RNA was removed using a RiboMinus Human/Mouse Transcriptome Isolation Kit (Invitrogen, Carlsbad, CA, USA). Using a random hexamer incorporating a T7 promoter, double-stranded cDNA was synthesized. Then cRNA, was generated from the double-stranded cDNA template through an in-vitro transcription reaction and purified using the Affymetrix sample clean-up module. Single-stranded cDNA was regenerated through a random-primed reverse transcription using a dNTP mix containing dUTP. The RNA was hydrolyzed with RNase H and the cDNA was purified. The cDNA was then fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide.

5.5 µg of the fragmented, biotinylated cDNA was added to a hybridization mixture, loaded on a Human Exon 1.0 ST GeneChip and hybridized for 16 hours at 45 °C and 60 rpm.

Using the Affymetrix exon array, genes are indirectly measured by exons analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using an Affymetrix GeneChip Scanner 3000, generating CEL files for each array.

Exon-level expression values were derived from the CEL file probe-level hybridization intensities using the model-based RMA algorithm as implemented in the Affymetrix Expression ConsoleTM software. RMA (Robust Multiarray Average) performs normalization, background correction and data summarization.
Differentially expressed genes between conditions are calculated using Anova (ANalysis Of Variance), a T-test for more than two groups.

The target identification is biassed since clinically well defined risk groups were analyzed. The markers are categorized based on their role in cancer biology. For the identification of markers different groups were compared: NPr with LG- and HG PrCa, PrCa Met with LG- and HG PrCa, CRPC with LG- and HG PrCa. Finally the samples were categorized based on clinical stage and organ confined PrCa (pT2) was compared with not-organ confined (pT3) PrCa.

Based on the expression analysis obtained, biomarkers were identified based on 99 prostate samples; the differences in expression levels between the different groups are provided in **Table 1 a,b,c and d.**

**Table 1a: Expression level differences between low grade (LG) - and high grade (HG) PrCa versus normal prostate (NPr) of 25 targets based on the analysis of 99 well annotated specimens**

| Gene Symbol | Gene Name | Gene assignment | Fold Change | LG + HG vs NPr | rank |
|---|---|---|---|---|---|
| CRISP3 | cysteine-rich secretory protein 3 | NM_006061 | 17,05 | up | 1 |
| GLYATL1 | glycine-N-acyltransferase-like 1 | NM_080661 | 10,24 | up | 2 |
| AMACR | alpha-methylacyl-CoA racemase | NM_014324 | 9,59 | up | 4 |
| TARP | TCR gamma alternate reading frame protein | NM_001003799 | 9,42 | up | 5 |
| ACSM1 | acyl-CoA synthetase medium-chain family member 1 | NM_052956 | 8,43 | up | 7 |
| TDRD1 | tudor domain containing 1 | NM_198795 | 7,70 | up | 8 |
| TMEM45B | transmembrane protein 45B | NM_138788 | 7,05 | up | 9 |
| FOLH1 | folate hydrolase (prostate-specific membrane antigen) 1 | NM_004476 | 6,47 | up | 10 |
| C19orf48 | chromosome 19 open reading frame 48 | NM_199249 | 5,91 | up | 12 |
| ALDH3B2 | aldehyde dehydrogenase 3 family, member B2 | NM_000695 | 5,66 | up | 13 |
| NETO2 | neuropilin (NRP) and tolloid (TLL)-like 2 | NM_018092 | 5,63 | up | 14 |
| MS4A8B | membrane-spanning 4-domains, subfamily A, member 8B | NM_031457 | 5,00 | up | 18 |
| TLCD1 | TLC domain containing 1 | NM_138463 | 4,73 | up | 21 |
| FASN | fatty acid synthase | NM_004104 | 4,69 | up | 22 |
| GRPR | gastrin-releasing peptide receptor | NM_005314 | 4,51 | up | 24 |
| HPN | hepsin | NM_182983 | 4,44 | up | 25 |
| PTPRT | protein tyrosine phosphatase, receptor type, T | NM_133170 | 4,21 | up | 29 |
| TOP2A | topoisomerase (DNA) II alpha 170kDa | NM_001067 | 3,79 | up | 37 |
| FAM111B | family with sequence similarity 111, member B | NM_198947 | 3,77 | up | 39 |
| NKAIN1 | Na+/K+ transporting ATPase interacting 1 | NM_024522 | 3,76 | up | 40 |
| DLX1 | distal-less homeobox 1 | NM_178120 | 3,54 | up | 52 |
| TPX2 | TPX2, microtubule-associated, homolog | NM_012112 | 3,47 | up | 54 |
| CGREF1 | cell growth regulator with EF-hand domain 1 | NM_006569 | 3,22 | up | 66 |
| DPT | Dermatopontin | NM_001937 | -6,31 | down | 2 |
| ASPA | aspartoacylase (Canavan disease) | NM_000049 | -5,17 | down | 7 |

**Table 1b: Expression level differences between prostate cancer metastasis (PrCa Met) versus low grade (LG)- and high grade (HG) PrCa of 11 targets based on the analysis of 99 well annotated specimens**

| Gene symbol | Gene Name | Gene assignment | Fold Change | PrCa Met vs LG + HG | rank |
|---|---|---|---|---|---|
| PPFIA2 | protein tyrosine phosphatase receptor type f interacting protein α2 | NM_003625 | 4,59 | up | 3 |
| CDC20 | cell division cycle 20 homolog (S. cerevisiae) | NM_001255 | 4,27 | up | 4 |
| FAM110B | family with sequence similarity 110, member B | NM_147189 | 3,70 | up | 6 |
| TARP | TCR gamma alternate reading frame protein | NM_001003799 | 3,26 | up | 12 |
| ANLN | anillin, actin binding protein | NM_018685 | 3,17 | up | 13 |
| KIF20A | kinesin family member 20A | NM_005733 | 3,16 | up | 14 |
| TPX2 | TPX2, microtubule-associated, homolog | NM_012112 | 3,15 | up | 15 |
| CDC2 | cell division cycle 2, G1 to S and G2 to M | NM_001130829 | 2,88 | up | 23 |
| PGM5 | phosphoglucomutase 5 | NM_021965 | -15,71 | down | 10 |
| MSMB | microseminoprotein, beta- | NM_002443 | -12,23 | down | 15 |
| HSPB8 | heat shock 22kDa protein 8 | NM_014365 | -12,10 | down | 16 |

**Table 1c: Expression level differences between CRPC versus low grade (LG) - and high grade (HG) PrCa of 21 targets based on the analysis of 99 well annotated specimens**

| Gene symbol | Gene Name | Gene assignment | Fold Change | CRPC vs LG + HG | rank |
|---|---|---|---|---|---|
| AR | androgen receptor | NM_000044 | 4,66 | up | 1 |
| UGT2B15 | UDP glucuronosyltransferase 2 family, polypeptide B15 | NM_001076 | 4,20 | up | 2 |
| CDC20 | cell division cycle 20 homolog (S. cerevisiae) | NM_001255 | 3,86 | up | 4 |
| TOP2A | topoisomerase (DNA) II alpha 170kDa | NM_001067 | 3,54 | up | 5 |
| MKI67 | antigen identified by monoclonal antibody Ki-67 | NM_002417 | 3,47 | up | 6 |
| TPX2 | TPX2, microtubule-associated, homolog | NM_012112 | 3,40 | up | 7 |
| AKR1C1 | aldo-keto reductase family 1, member C1 | NM_001353 | 3,35 | up | 8 |
| CDC2 | cell division cycle 2, G1 to S and G2 to M | NM_001130829 | 3,21 | up | 10 |
| ANLN | anillin, actin binding protein | NM_018685 | 3,08 | up | 11 |
| KIF4A | kinesin family member 4A | NM_012310 | 3,02 | up | 12 |
| PTTG1 | pituitary tumor-transforming 1 | NM_004219 | 2,95 | up | 13 |
| KIF20A | kinesin family member 2 | NM_005733 | 2,90 | up | 14 |
| AKR1C3 | aldo-keto reductase family 1, member C3 | NM_003739 | 2,88 | up | 15 |
| FAM111B | family with sequence similarity 111, member B | NM_198947 | 2,81 | up | 16 |
| CKS2 | CDC28 protein kinase regulatory subunit 2 | NM_001827 | 2,79 | up | 19 |
| UGT2B17 | UDP glucuronosyltransferase 2 family, polypeptide B17 | NM_ | 2,77 | up | 20 |
| BUB1 | budding uninhibited by benzimidazoles 1 homolog | NM_004336 | 2,75 | up | 21 |
| MSMB | microseminoprotein, beta- | NM_002443 | -6,99 | down | 6 |
| NR4A1 | nuclear receptor subfamily 4, group A, member 1 | NM_002135 | -6,57 | down | 7 |
| MT1M | metallothionein 1M | NM_176870 | -6,08 | down | 10 |
| DUSP1 | dual specificity phosphatase 1 | NM_004417 | -5,59 | down | 12 |

**Table 1d: Expression level differences between organ confined PrCa(pT2) versus not-organ confined (pT3)PrCa of 21 targets based on the analysis of 99 well annotated specimens**

| Gene symbol | Gene Name | Gene assignment | Fold Change | pT3 vs pT2 | rank |
|---|---|---|---|---|---|
| TTN | Titin | NM_133378 | 4,88 | up | 2 |
| SLN | Sarcolipin | NM_003063 | 3,62 | up | 3 |
| PPFIA2 | protein tyrosine phosphatase, receptor type f interacting protein alpha 2 | NM_003625 | 3,47 | up | 4 |
| COMP | cartilage oligomeric matrix protein | NM_000095 | 2,74 | up | 6 |
| ABI3BP | ABI family, member 3 | NM_015429 | 2,71 | up | 7 |
| NEB | Nebulin | NM_004543 | 2,58 | up | 8 |
| MT1M | metallothionein 1M | NM_176870 | -6,03 | down | 1 |
| MT1G | metallothionein 1G | NM_005950 | -3,61 | down | 2 |

As can be clearly seen in **table 1** an up or down regulation of expression of the shown genes was associated with prostate cancer, CRPC, prostate metastasis or tumor stage.

Considering the above results obtained in 99 tumor samples the expression data clearly demonstrates the suitable of these genes as biomarkers for the diagnosis of prostate cancer.

### Example 2

Using the gene expression profile (GeneChip® Human Exon 1.0 ST Array, Affymetrix) on 99 prostate samples several genes were found to be differentially expressed in normal prostate compared with prostate cancer and/or castration resistant prostate cancer(CRPC) or differentially expressed between low grade and high grade prostate cancer compared with CRPC and/or prostate cancer metastasis. Together with several other in the GeneChip® Human Exon 1.0 ST Array differentially expressed genes, the expression levels of these genes were validated using the TaqMan® Low Density arrays (TLDA, Applied Biosystems). In **Table 2** an overview of the validated genes is shown.

**Table 2: Gene expression assays used for TLDA analysis**

| Gene Symbol | Description | Gene-ID | amplicon size (bp) |
|---|---|---|---|
| ABI3BP | ABI family, member 3 (NESH) binding protein | NM_015429 | 84 |
| ACSM1 | acyl-CoA synthetase medium-chain family member 1 | NM_052956 | 74 |
| ALDH3B2 | aldehyde dehydrogenase 3 family, member B2 | NM_000695 | 126 |
| AMACR | alpha-methylacyl-CoA racemase | NM_014324 | 97 |
| ANLN | anillin, actin binding protein | NM_018685 | 71 |
| ASPA | aspartoacylase (Canavan disease) | NM_000049 | 63 |
| BUB1 | budding uninhibited by benzimidazoles 1 homolog | NM_004336 | 61 |
| C19orf48 | chromosome 19 open reading frame 48 | NM_199249 | 59 |
| CDC2 | cell division cycle 2, G1 to S and G2 to M | NM_033379 | 109 |
| CDC20 | cell division cycle 20 homolog (S. cerevisiae) | NM_001255 | 108 |
| CGREF1 | cell growth regulator with EF-hand domain 1 | NM_006569 | 58 |
| CKS2 | CDC28 protein kinase regulatory subunit 2 | NM_001827 | 73 |
| COMP | cartilage oligomeric matrix protein | NM_000095 | 101 |
| CRISP3 | cysteine-rich secretory protein 3 | NM_006061 | 111 |
| DLX1 | distal-less homeobox 1 | NM_178120 | 95 |
| DPT | Dermatopontin | NM_001937 | 67 |
| DUSP1 | dual specificity phosphatase 1 | NM_004417 | 63 |
| ERG | v-ets erythroblastosis virus E26 oncogene homolog | NM_004449 | 104 |
| FAM110B | family with sequence similarity 110, member B | NM_147189 | 74 |
| FAM111B | family with sequence similarity 111, member B | NM_198947 | 68 |
| FASN | fatty acid synthase | NM_004104 | 62 |
| FOLH1 | folate hydrolase (prostate-spec. membrane antigen) 1 | NM_004476 | 110 |
| GLYATL1 | glycine-N-acyltransferase-like 1 | NM_080661 | 83 |
| GRPR | gastrin-releasing peptide receptor | NM_005314 | 68 |
| HPRT1 | hypoxanthine phosphoribosyltransferase 1 | NM_000194 | 72 |
| HSPB8 | heat shock 22kDa protein 8 | NM_14365 | 66 |
| KIF20A | kinesin family member 20A | NM_005733 | 71 |
| KIF4A | kinesin family member 4A | NM_012310 | 88 |
| MKI67 | antigen identified by monoclonal antibody Ki-67 | NM_002417 | 66 |
| MS4A8B | membrane-spanning 4-domains, subfam. A, member 8B | NM_031457 | 62 |
| MSMB | microseminoprotein, beta- | NM138634 | 149 |
| MT1M | metallothionein 1M | NM_176870 | 144 |
| NETO2 | neuropilin (NRP) and tolloid (TLL)-like 2 | NM_018092 | 66 |
| NKAIN1 | Na+/K+ transporting ATPase interacting 1 | NM_024522 | 96 |
| NR4A1 | nuclear receptor subfamily 4, group A, member 1 | NM_002135 | 79 |
| PCA3 | prostate cancer antigen 3 | AF103907 | 52 |
| PGM5 | phosphoglucomutase 5 | NM_021965 | 121 |
| PPFIA2 | protein tyrosine phosphatase receptor type f polypept | NM_003625 | 66 |
| PTPRT | protein tyrosine phosphatase, receptor type, T | NM_133170 | 62 |
| PTTG1 | pituitary tumor-transforming 1 | NM_004219 | 86 |
| TDRD1 | tudor domain containing 1 | NM_198795 | 67 |
| ILCD1 | TLC domain containing 1 | NM_138463 | 63 |
| TMEM45B | transmembrane protein 45B | NM_138788 | 70 |
| TOP2A | topoisomerase (DNA) II alpha 170kDa | NM_001067 | 125 |
| TPX2 | TPX2, microtubule-associated, homolog | NM_012112 | 89 |
| TTN | Titin | NM_133378 | 85 |
| UGT2B15 | UDP glucuronosyltransferase 2 family,polypeptide B15 | NM_001076 | 148 |

Prostate samples in the following categories were used:
- Normal prostate (NPr) (n=6)
- Benign Prostatic Hyperplasia (BPH)(n=6)
- Low grade prostate cancer (LG PrCa)(n=14): tissue specimens from primary tumors with a Gleason Score ≤6 obtained after radical prostatectomy. This group represents patients with a good prognosis.
- High grade prostate cancer (HG PrCa)(n=14): tissue specimens from primary tumors with a Gleason Score ≥7 obtained after radical prostatectomy. This group represents patients with poor prognosis.
- Castration resistant prostate cancer (CRPC)(n=14): tissue specimens are obtained from patients that are progressive under endocrine therapy and who underwent a transurethral resection of the prostate (TURP).
- Prostate cancer metastases (PrCa Met)(n=8): tissue specimens are obtained from positive lymfnodes after LND or after autopsy. This group represents patients with poor prognosis
All tissue samples were snap frozen and cryostat sections were stained with hematoxylin and eosin (H.E.). These H.E.-stained sections were classified by a pathologist.

Tumor- and tumor free areas were dissected. RNA was extracted from 10 µm thick serial sections that were collected from each tissue specimen at several levels. Tissue was evaluated by HE-staining of sections at each level and verified microscopically. Total RNA was extracted with TRIzol® (Invitrogen, Carlsbad, CA, USA) according to the manufacturer's instructions.
RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg DNase-treated total RNA was reverse transcribed using SuperScript™ II Reverse Transcriptase (Invitrogen) in a 37.5 µl reaction according to the manufacturer's protocol. Reactions were incubated for 10 minutes at 25°C, 60 minutes at 42°C and 15 minutes at 70°C. To the cDNA, 62.5 µl milliQ was added.

For the validation not only prostate tissue specimens were used. To investigate whether the selected markers could successfully be detected in body fluids also normal bladder tissue specimens, peripheral blood lymphocytes(PBL)- and urinary sediment specimens were included in the marker validation step. The background signal of the markers in normal bladder and urinary sediments from patients without prostate cancer should be low.

These urinary sediment specimens were collected at three hospitals after a consent form approved by the institutional review board was signed by all participants. First voided urine samples were collected after digital rectal examination (DRE) from men scheduled for prostate cancer. After urine specimen collection, the urologist performed prostate biopsies according to a standard protocol. Prostate biopsies were evaluated and in case prostate cancer was present the Gleason score was determined.

First voided urine after DRE (20-30 ml) was collected in a coded tube containing 2 ml 0.5M EDTA pH 8.0. All samples were immediately cooled to 4°C and were mailed in batches with cold packs to the laboratory of NovioGendix. The samples were processed within 48 h after the samples was acquired to guarantee good sample quality. Upon centrifugation at 4°C and 1,800 x g for 10 minutes, urinary sediments were obtained. These urinary sediments were washed twice with ice-cold buffered sodium chloride solution (at 4°C and 1,800 x g for 10 minutes), snap-frozen in liquid nitrogen, and stored at -70°C.

Total RNA was extracted from these urinary sediments, using TriPure Isolation Reagent (Roche Diagnostics, Almere, the Netherlands) according to the manufacturers protocol.

Two additional steps were added. First 2 µl glycogen (15 mg/ml) was added as a carrier (Ambion, Austin (TX), USA) before precipitation with isopropanol. Secondly a second precipitation step with 3M sodium-acetate pH 5.2 and 100% ethanol was performed to discard traces of TriPure Isolation Reagent.

The RNA was dissolved in RNase-free water and incubated for 10 minutes at 55-60°C. The RNA was DNase treated using amplification grade DNaseI (Invitrogen™, Breda, the Netherlands) according to the manufacturers protocol. Again glycogen was added as carrier and the RNA was precipitated with 3M sodium-acetate pH 5.2 and 100% ethanol for 2hr at -20°C.

After removing the last traces of ethanol, the RNA pellet was dissolved in 16.5 µl RNase-free water. The RNA concentration was determined through OD-measurement (Nanodrop) and 1 µg of total RNA was used for RNA amplification using the Ambion®WT Expression Kit (Ambion, Austin (TX), USA) according to the manufacturers protocol.

To determine gene expressions levels the cDNA generated from RNA extracted from both the tissue specimens and the urinary sediments was used as template in TaqMan® Low Density Arrays (TLDA; Applied Biosystems).

A list of assays used in this study is given in **Table 2.** Of the individual cDNAs, 5 µl is added to 50 µl Taqman® Universal Probe Master Mix (Applied Biosystems)and 50 µl milliQ. One hundred µl of each sample was loaded into 1 sample reservoir of a TaqMan® Array (384-Well Micro Fluidic Card) (Applied Biosystems). The TaqMan® Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of hypoxanthine phosphoribosyltransferase 1 (HPRT) (endogenous control gene).

Furthermore, gene expression values were calculated based on the comparative threshold cycle (Ct) method, in which a normal prostate RNA sample was designated as a calibrator to which the other samples were compared.

For the validation of the differentially expressed genes found by the GeneChip® Human Exon 1.0 ST Array, 60 prostate tissue specimens were used in TaqMan® Low Density arrays (TLDAs). To investigate whether the markers might be used in body fluids also 2 normal bladder tissue specimens, 2 peripheral blood lymphocyte specimens and 16 urinary sediments (from which 9 had PrCa in their biopsies and 7 did not) were included.

In the TLDAs, expression levels were determined for the 47 genes of interest. The prostate cancer specimens were put in order from normal prostate, BPH, low Gleason scores, high Gleason scores, CRPC and finally prostate cancer metastasis. Both GeneChip® Human Exon 1.0 ST Array and TLDA data were analyzed using scatter- and box plots.

After analysis of the box- and scatterplots a list of suitable genes indicative for prostate cancer and the prognosis thereof was obtained (**Table 3,** **Figures 14** **t/m 26**).

**Table 3: List of genes identified**

| Gene Symbol | Gene description | Up/down in group | Rank | Gene-ID |
|---|---|---|---|---|
| ACSM1 | acyl-CoA synthetase medium-chain family member 1 | Up in LG/HG vs NPr | 7 | NM_052956 |
| ALDH3B2 | aldehyde dehydrogenase 3 family, member B2 | Up in LG/HG vs NPr | 13 | NM_000695 |
| CGREF1 | cell growth regulator with EF-hand domain 1 | Up in LG/HG vs NP | 66 | NM_006569 |
| COMP | cartilage oligomeric matrix protein | Up in pT3 vs pT2 | 6 | NM_000095 |
| C19orf48 | chromosome 19 open reading frame 48 | Up in LG/HG vs NPr | 12 | NM_199249 |
| DLX1 | distal-less homeobox 1 | Up in LG/HG vs NPr | 52 | NM_178120 |
| GLYATL1 | glycine-N-acyltransferase-like 1 | Up in LG/HG vs NPr | 1 | NM_080661 |
| MS4A8B | membrane-spanning 4-domains,subfam. A, member 8B | Up in LG/HG vs NPr | 18 | NM_031457 |
| NKAIN1 | Na+/K+transporting ATPase interacting 1 | Up in LG/HG vs NPr | 40 | NM_024522 |
| PPFIA2 | protein tyrosine phosphatase,receptor type,f polypept. (PTPRF) | Up in Meta vs LG/HG | 3 | NM_003625 |
| | | Up in pT3 vs pT2 | 4 | |
| PTPRT | protein tyrosine phosphatase receptor type T | Up in LG/HG vs NPr | 29 | NM_133170 |
| TDRD1 | tudor domain containing 1 | Up in LG/HG vs NPr | 8 | NM_198795 |
| UGT2B15 | UDP glucuronosyltransferase 2 family, polypeptide B15 | Up in CRPC vs LG/HG | 2 | NM 001076 |

ACSM1 (**Figure 14**): The present GeneChip® Human Exon 1.0 ST Array data showed that ACSM1 was upregulated in the groups LG PrCa, HG PrCa, CRPC and PrCa Met compared to NPr and BPH. Validation experiments using TaqMan® Low Density arrays confirmed this upregulation in PrCa. Therefore, ACSM1 has diagnostic potential.

The expression of ACSM1 in normal bladder and PBL is very low. Furthermore, the expression of ACSM1 in urinary sediments obtained from patients with PrCa is higher compared to its expression in urinary sediments obtained from patients without PrCa. Therefore, ACSM1 has diagnostic potential as a urinary marker for prostate cancer.

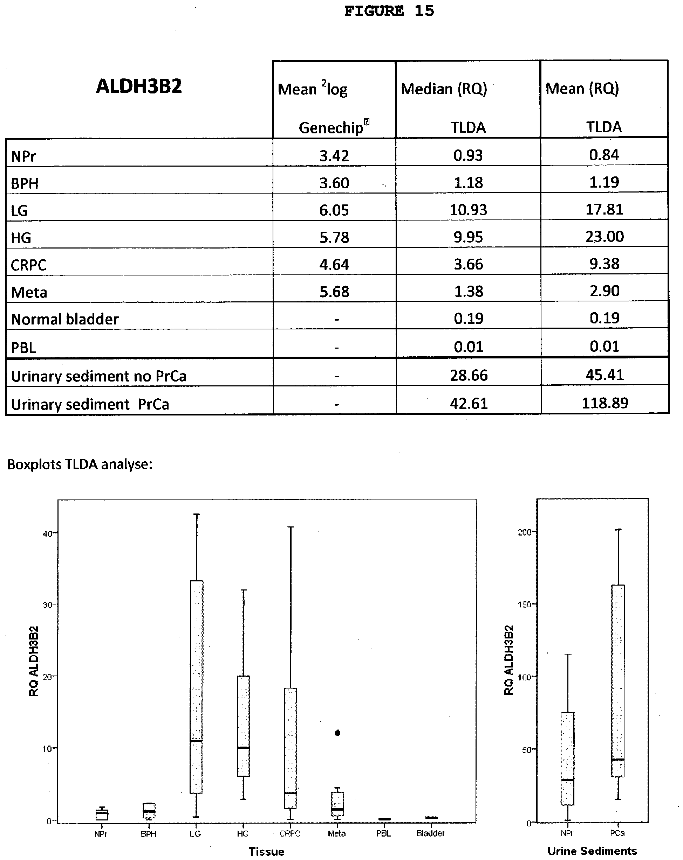

ALDH3B2(**Figure 15**): The present GeneChip® Human Exon 1.0 ST Array data showed that ALDH3B2 was upregulated in the groups LG PrCa, HG PrCa, CRPC and PrCa Met compared to NPr and BPH. Validation experiments using TaqMan® Low Density arrays confirmed the upregulation in these groups with exception of PrCa Met. Therefore, ALDH3B2 has diagnostic potential.

The expression of ALDH3B2 in normal bladder and PBL is very low. Furthermore, the expression of ALDH3B2 in urinary sediments obtained from patients with PrCa is higher compared to its expression in urinary sediments obtained from patients without PrCa. Therefore, ALDH3B2 has diagnostic potential as a urinary marker for prostate cancer.

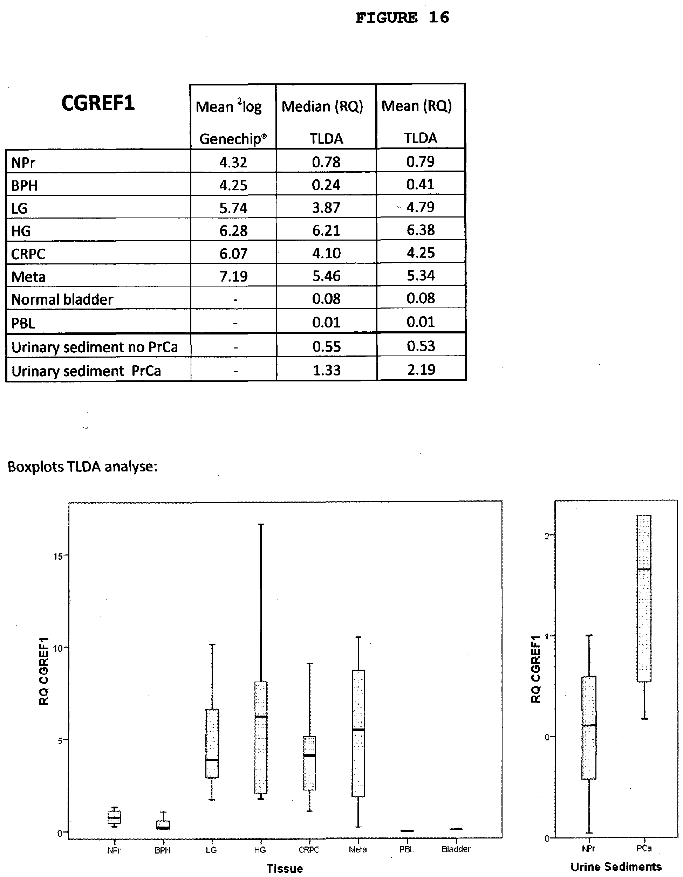

CGREF1 (**Figure 16**): The present GeneChip® Human Exon 1.0 ST Array data showed that CGREF1 was upregulated in the groups LG PrCa, HG PrCa, CRPC and PrCa Met compared to NPr and BPH. Validation experiments using TaqMan® Low Density arrays confirmed this upregulation. Therefore, CGREF1 has diagnostic potential.

The expression of CGREF1 in normal bladder and PBL is very low. Furthermore, the expression of CGREF1 in urinary sediments obtained from patients with PrCa is higher (almost two separate groups)compared to its expression in urinary sediments obtained from patients without PrCa. Therefore, CGREF1 has diagnostic potential as a urinary marker for prostate cancer.

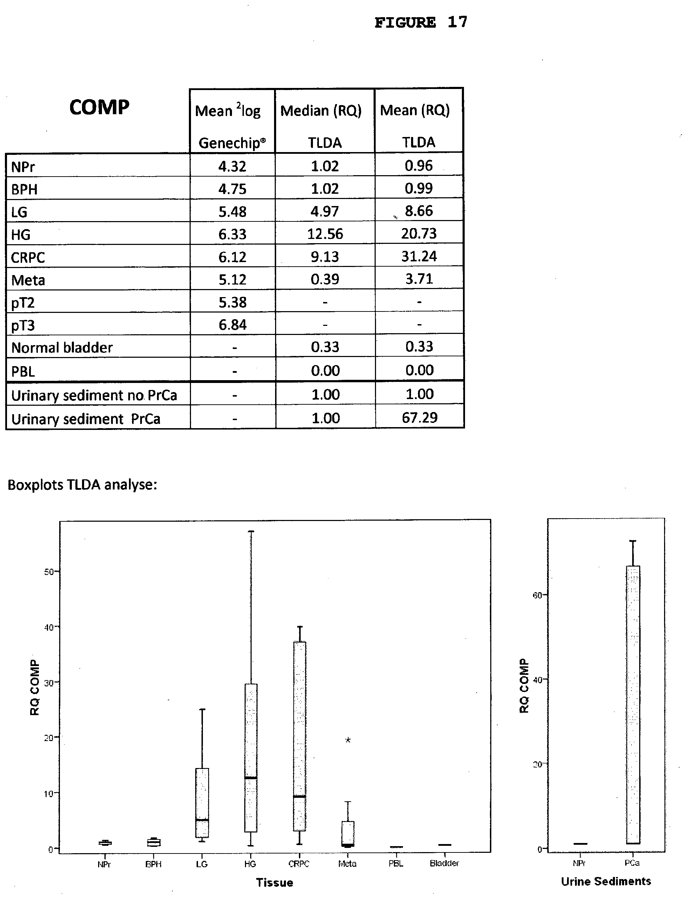

COMP(**Figure 17**): The present GeneChip® Human Exon 1.0 ST Array data showed that COMP was upregulated (up to 3.5 fold) in the groups LG PrCa, HG PrCa, CRPC and PrCa Met compared to NPr and BPH. Validation experiments using TaqMan® Low Density arrays confirmed this and showed an even larger upregulation in PrCa versus NPr tissue (up to 32.5 fold).Therefore, we conclude that COMP has diagnostic potential.

The expression of COMP in normal bladder and PBL is very low to undetectable levels. Furthermore, the expression of COMP in urinary sediments obtained from patients with PrCa is higher compared to its expression in urinary sediments obtained from patients without PrCa. Therefore, COMP has diagnostic potential as a urinary marker for prostate cancer.

The expression of COMP in locally advanced PrCa (pT3) is higher than in organ confined PrCa (pT2). Therefore, COMP can be used as a prognostic marker for prostate cancer (GeneChip® data).

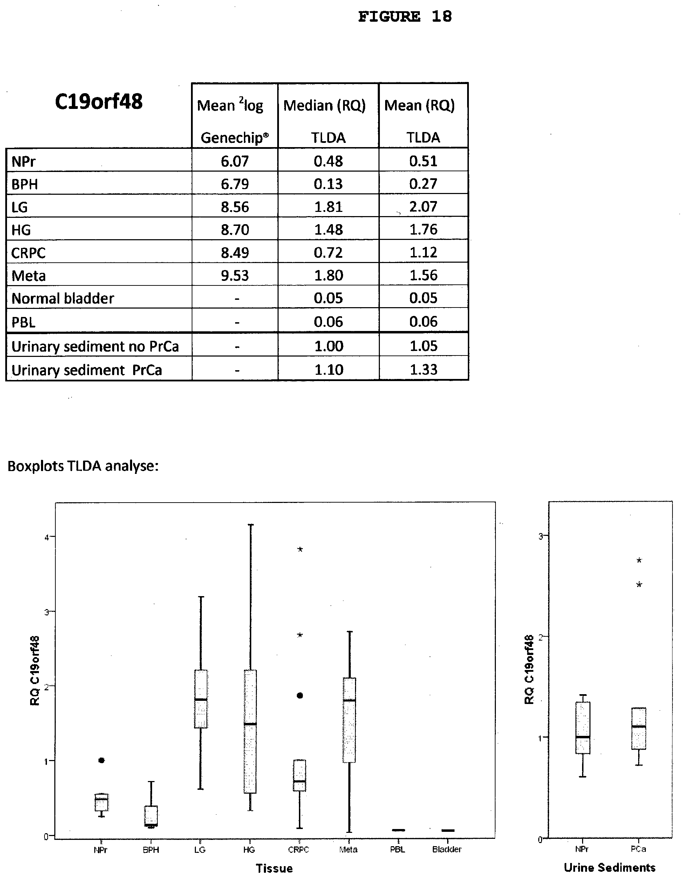

C19orf48(**Figure 18**): The present GeneChip® Human Exon 1.0 ST Array data showed that C19orf48 was upregulated in the groups LG PrCa, HG PrCa, CRPC and PrCa Met compared to NPr and BPH. Validation experiments using TaqMan® Low Density arrays confirmed this upregulation. Therefore, C19orf48 has diagnostic potential.

The expression of C19orf48 in normal bladder and PBL is very low. The mean expression of C19orf48 in urinary sediments obtained from patients with PrCa is not higher compared to its expression in urinary sediments obtained from patients without PrCa. However, in two out of nine urinary sediments obtained from patients with PrCa the expression is extremely higher (stars in boxplot)and these two patients would not be detected by most other biomarkers. Therefore, C19orf48 has complementary diagnostic potential as a urinary marker for prostate cancer.

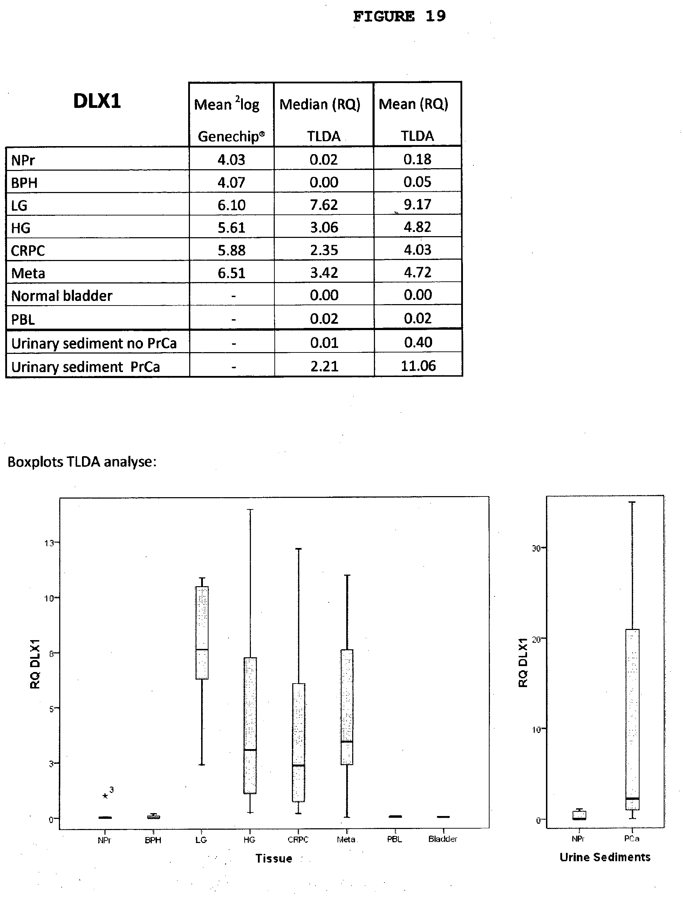

DLX1(**Figure 19**): The present GeneChip® Human Exon 1.0 ST Array data showed that DLX1 was upregulated (up to 5.6-fold)in the groups LG PrCa, HG PrCa, CRPC and PrCa Met compared to NPr and BPH.

Validation experiments using TaqMan® Low Density arrays confirmed this and showed an even larger upregulation in PrCa versus NPr tissue (up to 183.4 fold). Therefore, DLX1 has diagnostic potential.

The expression of DLX1 in normal bladder and PBL is undetectable to very low. Furthermore, the expression of DLX1 in urinary sediments obtained from patients with PrCa is much higher compared to its expression in urinary sediments obtained from patients without PrCa. Therefore, DLX1 has diagnostic potential as a urinary marker for prostate cancer.

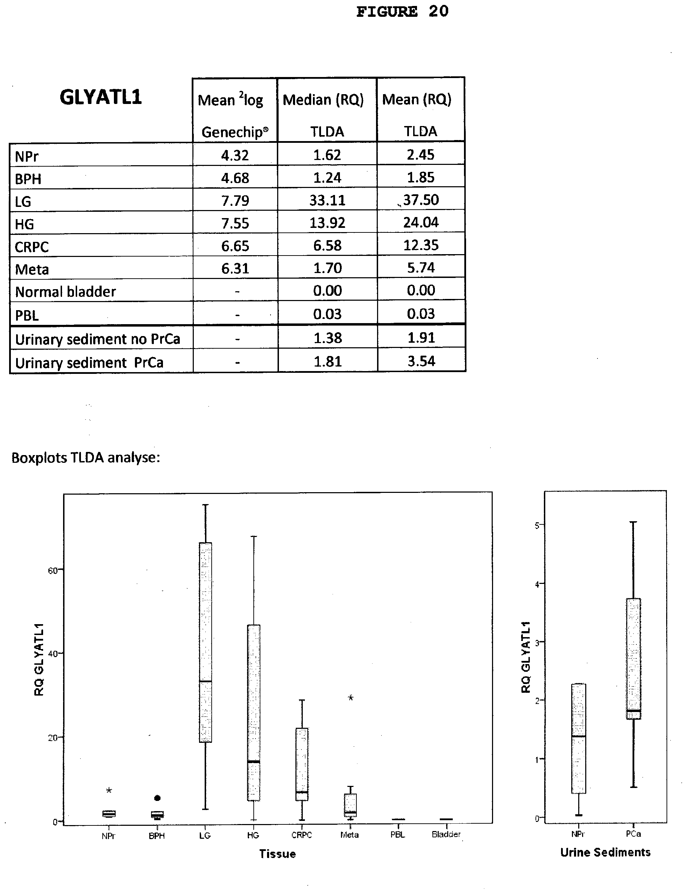

GLYATL1(**Figure 20**): The present GeneChip® Human Exon 1.0 ST Array data showed that GLYATL was upregulated in the groups LG PrCa, HG PrCa, CRPC and PrCa Met compared to NPr and BPH. Validation experiments using TaqMan® Low Density arrays confirmed this. Therefore, GLYATL has diagnostic potential.

The expression of GLYATL1 in normal bladder and PBL is undetectable to very low. Furthermore, the expression of GLYATL1 in urinary sediments obtained from patients with PrCa is higher compared to its expression in urinary sediments obtained from patients without PrCa. Therefore, GLYATL1 has diagnostic potential as a urinary marker for prostate cancer.

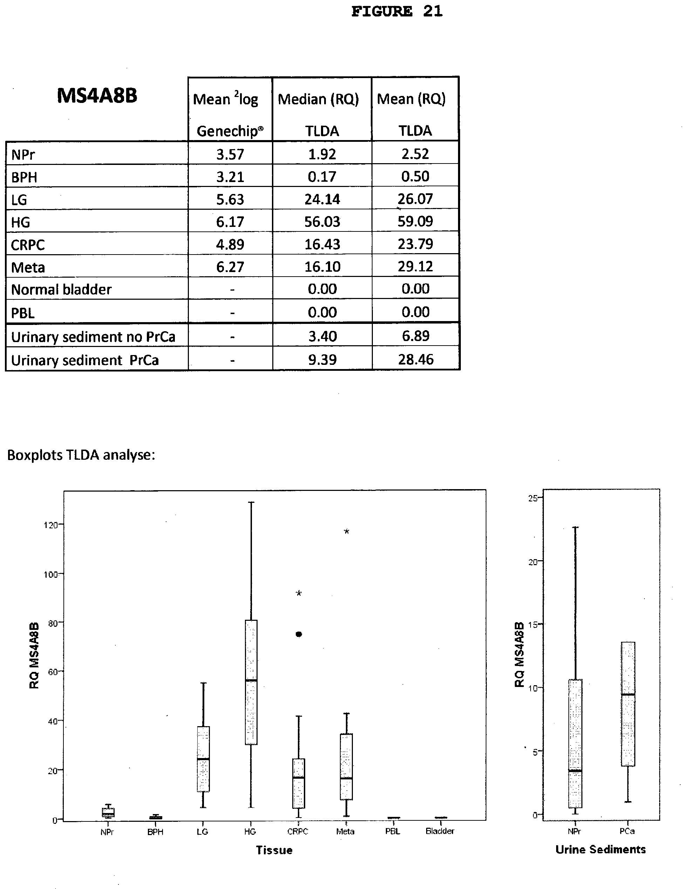

MS4A8B(**Figure 21**): The present GeneChip® Human Exon 1.0 ST Array data showed that MS4A8B was upregulated in LG PrCa, HG PrCa, CRPC and PrCa Met (up to 8.3 fold) compared to NPr and BPH. Validation experiments using TaqMan® Low Density arrays confirmed this and showed an even larger upregulation in PrCa versus NPr tissue (up to 119.8 fold). Therefore, MS4A8B has diagnostic potential.

The expression of MS4A8B in normal bladder and PBL is undetectable. Furthermore, the expression of MS4A8B in urinary sediments obtained from patients with PrCa is higher compared to its expression in urinary sediments obtained from patients without PrCa. Therefore, MS4A8B has diagnostic potential as a urinary marker for prostate cancer.

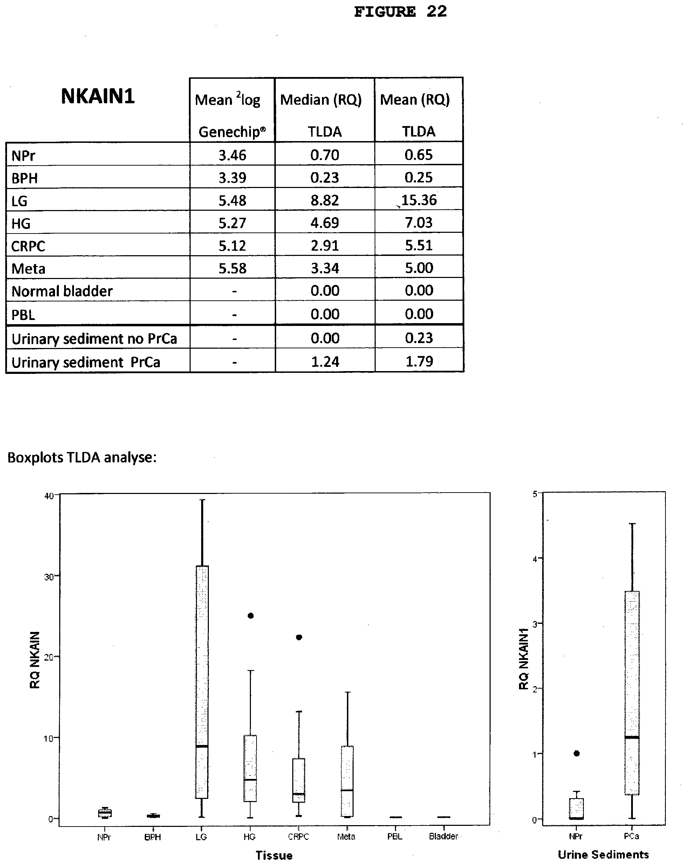

NKAIN1(**Figure 22**): The present GeneChip® Human Exon 1.0 ST Array data showed that NKAIN1 was upregulated in LG PrCa, HG PrCa, CRPC and PrCa Met(up to 4.6 fold) compared to NPr and BPH. Validation experiments using TaqMan® Low Density arrays confirmed this and showed an even larger upregulation in PrCa versus NPr tissue (up to 61.4 fold). Therefore, NKAIN1 has diagnostic potential.

The expression of NKAIN1 in normal bladder and PBL is undetectable. Furthermore, the expression of NKAIN1 in urinary sediments obtained from patients with PrCa is higher(almost two separate groups in boxplot)compared to its expression in urinary sediments obtained from patients without PrCa. Therefore, NKAIN1 diagnostic potential as a urinary marker for prostate cancer.

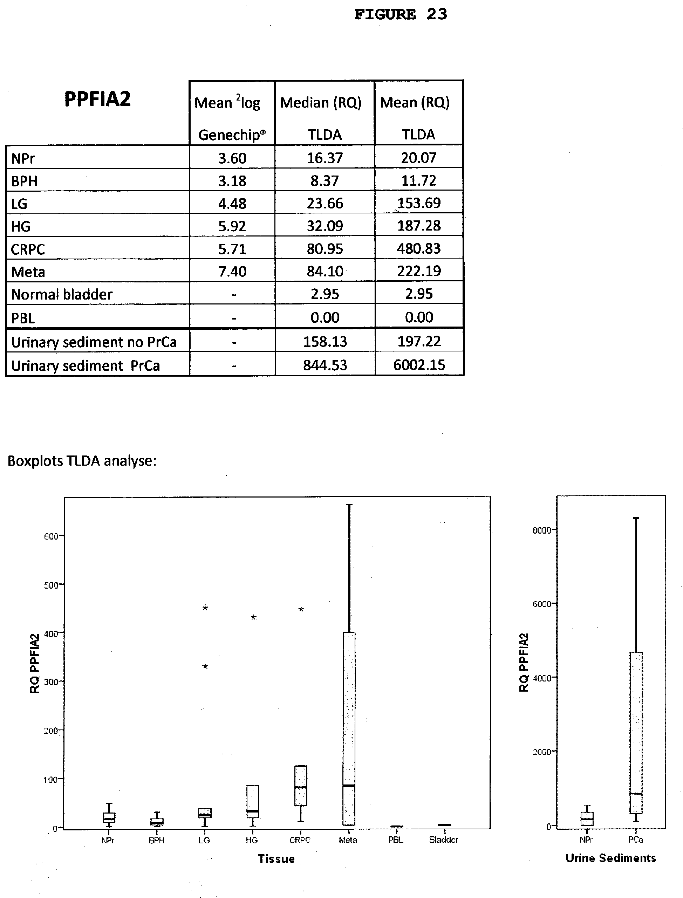

PPFIA2(**Figure 23**): The present GeneChip® Human Exon 1.0 ST Array data showed that PPFIA2 was upregulated in LG PrCa, HG PrCa, CRPC and PrCa Met compared to NPr and BPH. This upregulation was highest in PrCa Met.

Validation experiments using TaqMan® Low Density arrays confirmed the upregulation in these groups. Therefore, PPFIA2 has diagnostic potential

The expression of PPFIA2 in normal bladder and PBL is low to undetectable. Furthermore, the expression of PPFIA2 in urinary sediments obtained from patients with PrCa is much higher (almost two separate groups in boxplot) compared to its expression in urinary sediments obtained from patients without PrCa. Therefore, PPFIA2 has diagnostic potential as a urinary marker for prostate cancer.

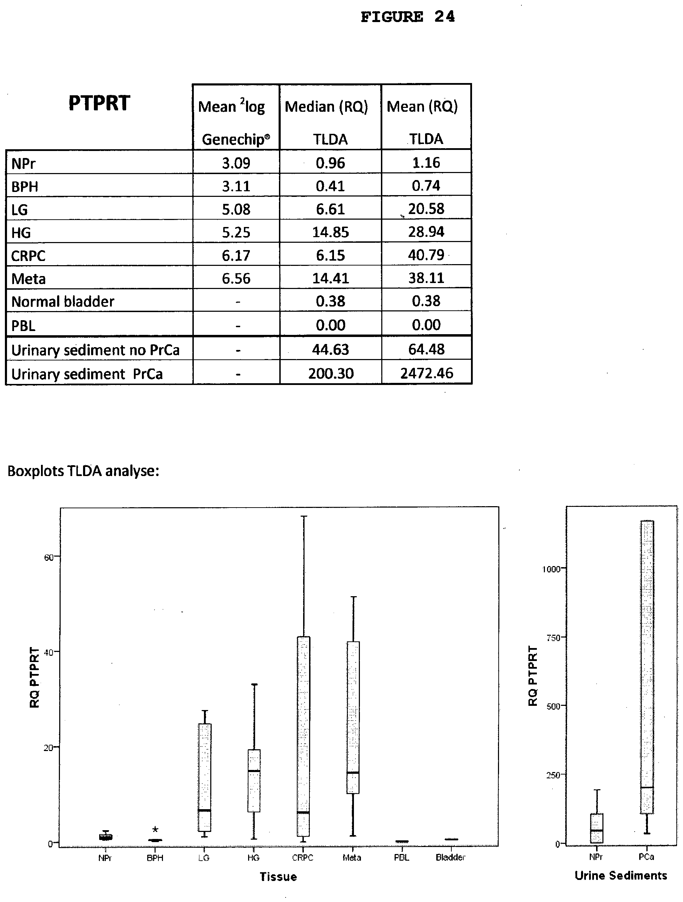

PTPRT(**Figure 24**): The present GeneChip® Human Exon 1.0 ST Array data showed that PTPRT was upregulated (up to 11.1 fold) in the groups LG PrCa, HG PrCa, CRPC and PrCa Met compared to NPr and BPH. Validation experiments using TaqMan® Low Density arrays confirmed this and showed an even larger upregulation in PrCa versus NPr tissue (up to 55.1 fold). Therefore, PTPRT has diagnostic potential.

The expression of PTPRT in normal bladder and PBL is very low to undetectable. Furthermore, the expression of PTPRT in urinary sediments obtained from patients with PrCa is much higher (almost two separate groups in boxplot)compared to its expression in urinary sediments obtained from patients without PrCa. Therefore, PTPRT has diagnostic potential as a urinary marker for prostate cancer.

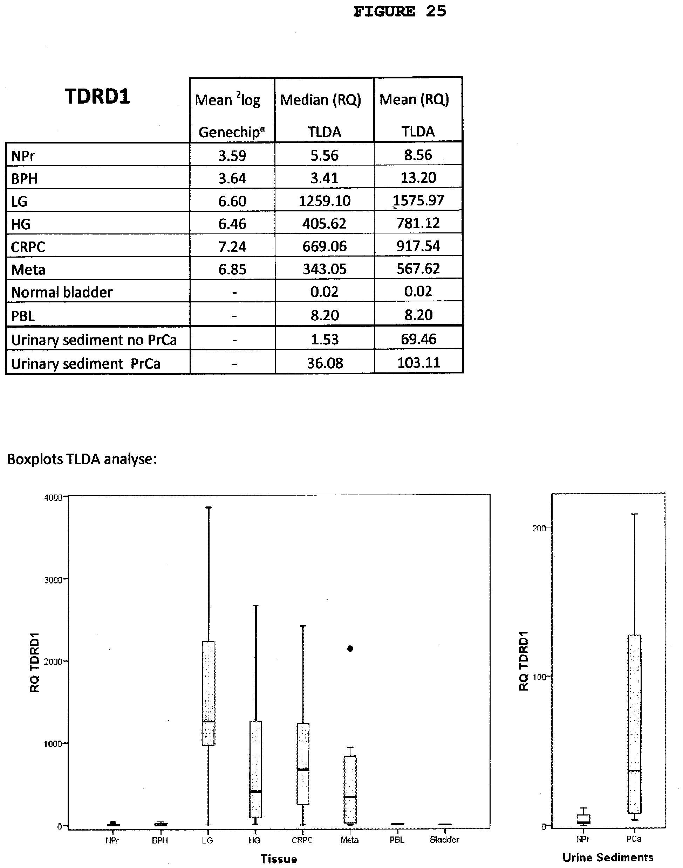

TDRD1 **(****Figure 25**): The present GeneChip® Human Exon 1.0 ST Array data showed that TDRD1 was upregulated (up to 12.6 fold) in the groups LG PrCa, HG PrCa, CRPC and PrCa Met compared to NPr and BPH. Validation experiments using TaqMan® Low Density arrays confirmed this and showed an even larger upregulation in PrCa versus NPr tissue (up to 184.1 fold), especially in the group of LG PrCa. Therefore, TDRD1 has diagnostic potential.

The expression of TDRD1 in normal bladder is very low. Furthermore, the expression of TDRD1 in urinary sediments obtained from patients with PrCa is much higher (two separate groups in boxplot)compared to its expression in urinary sediments obtained from patients without PrCa. Therefore, TDRD1 has diagnostic potential as a urinary marker for prostate cancer.

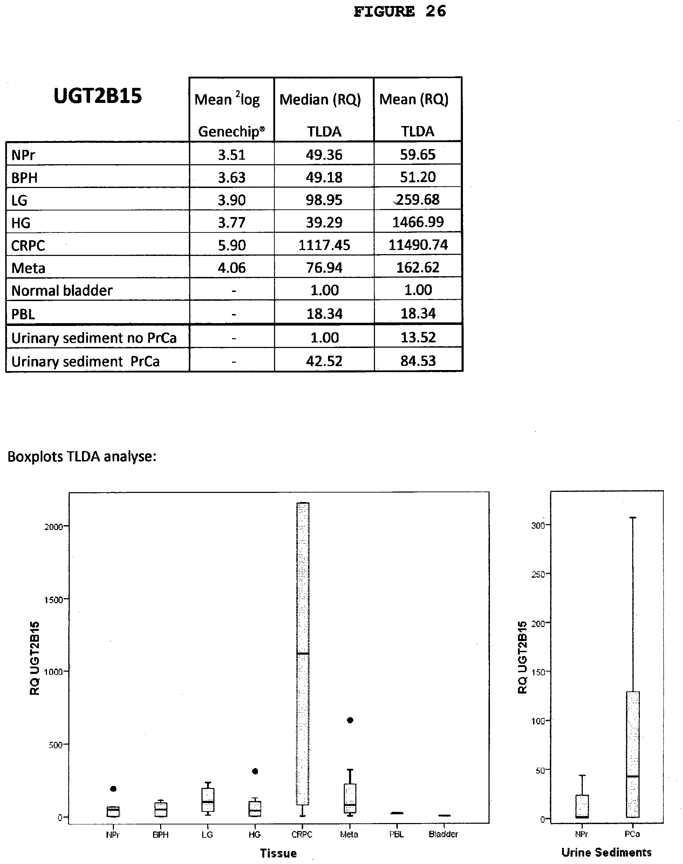

UGT2B15 **(****Figure 26**): The present GeneChip® Human Exon 1.0 ST Array data showed that UGT2B15 was upregulated (up to 5.2 fold) in the groups LG PrCa, HG PrCa, CRPC and PrCa Met compared to NPr and BPH. Validation experiments using TaqMan® Low Density arrays confirmed this and showed an even larger upregulation in PrCa versus NPr tissue (up to 224.4 fold). The expression of UGT2B15 in normal bladder is very low. Furthermore, the expression of UGT2B15 in urinary sediments obtained from patients with PrCa is higher compared to its expression in urinary sediments obtained from patients without PrCa. Therefore, UGT2B15 has diagnostic potential as a urinary marker for prostate cancer.

Since UGT2B15 is highly upregulated in CRPC patients, it is a suitable marker to monitor patients who undergo hormonal therapy for their locally advanced prostate cancer. Therefore, UGT2B15 has also prognostic value.

### Example 2

To identify markers for aggressive prostate cancer, the gene expression profile (GeneChip® Human Exon 1.0 ST Array, Affymetrix) of samples from patients with prostate cancer in the following categories were used:
- LG: low grade PrCa (Gleason Score equal or less than 6). This group represents patients with good prognosis;
- HG: high grade PrCa (Gleason Score of 7 or more). This group represents patients with poor prognosis; sample type, mRNA from primary tumor;
- PrCa Met. This group represents patients with poor prognosis; sample type; mRNA from PrCa metastasis;
- CRPC: castration resistant prostate cancer; mRNA from primary tumor material from patients that are progressive under endocrine therapy. This group represents patients with aggressive localized disease.

The expression analysis is performed according to standard protocols. Briefly, from patients with prostate cancer (belonging to one of the four previously mentioned categories) tissue was obtained after radical prostatectomy or TURP. The tissues were snap frozen and cryostat sections were H.E. stained for classification by a pathologist.

Tumor areas were dissected and total RNA was extracted with TRIzol (Invitrogen, Carlsbad, CA, USA) following manufacturer's instructions. The total RNA was purified with the Qiagen RNeasy mini kit (Qiagen, Valencia, CA, USA). Integrity of the RNA was checked by electrophoresis using the Agilent 2100 Bioanalyzer.

From the purified total RNA, 1 µg was used for the GeneChip Whole Transcript (WT) Sense Target Labeling Assay (Affymetrix, Santa Clara, CA, USA). According to the protocol of this assay, the majority of ribosomal RNA was removed using a RiboMinus Human/Mouse Transcriptome Isolation Kit (Invitrogen, Carlsbad, CA, USA). Using a random hexamer incorporating a T7 promoter, double-stranded cDNA was synthesized. Then cRNA, was generated from the double-stranded cDNA template through an in-vitro transcription reaction and purified using the Affymetrix sample clean-up module. Single-stranded cDNA was regenerated through a random-primed reverse transcription using a dNTP mix containing dUTP. The RNA was hydrolyzed with RNase H and the cDNA was purified. The cDNA was then fragmented by incubation with a mixture of UDG (uracil DNA glycosylase) and APE1 (apurinic/apyrimidinic endonuclease 1) restriction endonucleases and, finally, end-labeled via a terminal transferase reaction incorporating a biotinylated dideoxynucleotide.

5.5 µg of the fragmented, biotinylated cDNA was added to a hybridization mixture, loaded on a Human Exon 1.0 ST GeneChip and hybridized for 16 hours at 45 °C and 60 rpm.

Using the GeneChip® Human Exon 1.0 ST Array (Affymetrix), genes are indirectly measured by exons analysis which measurements can be combined into transcript clusters measurements. There are more than 300,000 transcript clusters on the array, of which 90,000 contain more than one exon. Of these 90,000 there are more than 17,000 high confidence (CORE) genes which are used in the default analysis. In total there are more than 5.5 million features per array.

Following hybridization, the array was washed and stained according to the Affymetrix protocol. The stained array was scanned at 532 nm using an Affymetrix GeneChip Scanner 3000, generating CEL files for each array.

Exon-level expression values were derived from the CEL file probe-level hybridization intensities using the model-based RMA algorithm as implemented in the Affymetrix Expression ConsoleTM software. RMA (Robust Multiarray Average) performs normalization, background correction and data summarization. Differentially expressed genes between conditions are calculated using Anova (ANalysis Of Variance), a T-test for more than two groups.

The target identification is biased since clinically well defined risk groups were analyzed. The markers are categorized based on their role in cancer biology. For the identification of markers the PrCa Met group is compared with 'HG' and 'LG'.

Based on the expression analysis obtained, biomarkers were identified based on 30 tumors; the expression profiles of the biomarkers are provided in **Table 4.**

**Table 4: Expression characteristics of 7 targets characterizing the aggressive metastatic phenotype of prostate cancer based on the analysis of 30 well annotated specimens**

| **Gene name** | **Gene assignment** | **Expression in PrCa Met** | **Met-LG** | **Rank** | **Met-HG** | **Rank** | **Met-CRPC** |
|---|---|---|---|---|---|---|---|
| PTPR | NM_003625 | Up | 15.89 | 4 | 8.28 | 4 | 11.63 |
| EPHA6 | NM_001080448 | Up | 15.35 | 5 | 9.25 | 2 | 8.00 |
| Plakophilin 1 | NM_000299 | Up | 5.28 | 28 | 4.92 | 8 | 5.46 |
| HOXC6 | NM_004503 | Up | 5.35 | 27 | 3.34 | 43 | 3.51 |
| HOXD3 | NM_006898 | Up | 1.97 | 620 | 2.16 | 238 | 1.40 |
| sFRP2 | NM_003013 | Down | -6.06 | 102 | -13.93 | 15 | -3.53 |
| HOXD10 | NM_002148 | Down | -3.71 | 276 | -3.89 | 238 | -5.28 |

### Example 3

The protocol of example 1 was repeated on a group of 70 specimens. The results obtained are presented in **Table 5.**

**Table 5: Expression characteristics of 7 targets validated in the panel of 70 tumors**

| **Gene name** | **Gene assignment** | **Expression in PrCa met** | **Met-LG** | **Rank** | **Met-HG** | **Rank** | **Met-CRPC** | **Rank** |
|---|---|---|---|---|---|---|---|---|
| PTPR | NM_003625 | Up | 6,92 | 1 | 2,97 | 11 | 3,66 | 2 |
| EPHA6 | NM_001080448 | Up | 4,35 | 4 | 3,97 | 3 | 3,18 | 3 |
| Plakophilin 1 | NM_000299 | Up | 3,18 | 12 | 4,00 | 2 | 4,11 | 5 |
| HOXC6 | NM_004503 | Up | 1,77 | 271 | 1,75 | 208 | 1,44 | 6 |
| HOXD3 | NM_006898 | Up | 1,62 | 502 | 1,66 | 292 | 1,24 | 7 |
| sFRP2 | NM_003013 | Down | -6,28 | 46 | -10,20 | 10 | -5,86 | 1 |
| HOXD10 | NM_002148 | Down | -2,48 | 364 | -2,55 | 327 | -2,46 | 4 |

As can be clearly seen in **Tables 4** and **5,** an up regulation of expression of PTPR, EPHA6, Plakophilin 1, HOXC6 (**Figure 27**) and HOXD3 was associated with prostate cancer. Further, as can be clearly seen in **Tables 4** and **5,** a down-regulation of expression of sFRP2 (**Figure 28**) and HOXD10 (**Figure 29**) was associated with prostate cancer.

Considering the above results obtained in 70 tumour samples, the expression data clearly demonstrates the suitability of these genes as bio- or molecular marker for the diagnosis of prostate cancer.

### Example 4

Using the gene expression profile (GeneChip® Human Exon 1.0 ST Array, Affymetrix) on 70 prostate cancers several genes were found to be differentially expressed in low grade and high grade prostate cancer compared with prostate cancer metastasis and castration resistant prostate cancer (CRPC). Together with several other in the GeneChip® Human Exon 1.0 ST Array differentially expressed genes, the expression levels of these genes were validated using the TaqMan® Low Density arrays (TLDA, Applied Biosystems). In **Table 6** an overview of the validated genes is shown.

**Table 6: Gene expression assays used for TLDA analysis**

| **Symbol** | **Gene description** | **Accession number** | **Amplicon size** |
|---|---|---|---|
| AMACR | alpha-methylacyl-CoA racemase | NM_014324 | 97-141 |
| B2M | Beta-2-microglobulin | NM_004048 | 64-81 |
| CYP4F8 | cytochrome P450, family 4, subfamily F | NM_007253 | 107 |
| CDH1 | E-Cadherin | NM_004360 | 61-80 |
| EPHA6 | ephrin receptor A6 | NM_001080448 | 95 |
| ERG | v-ets erythroblastosis virus E26 oncogene homolog | NM_004449 | 60-63 |
| ETV1 | ets variant 1 | NM_004956 | 74-75 |
| ETV4 | ets variant 4 | NM_001986 | 95 |
| ETV5 | ets variant 5 | NM_004454 | 70 |
| FASN | fatty acid synthase | NM_004104 | 144 |
| FOXD1 | forkhead box D1 | NM_004472 | 59 |
| HOXC6 | homeobox C6 | NM_004503 | 87 |
| HOXD3 | homeobox D3 | NM_006898 | 70 |
| HOXD10 | homeobox D10 | NM_002148 | 61 |
| HPRT | hypoxanthine phosphoribosyltransferase 1 | NM_000194 | 72-100 |
| HSD17B6 | hydroxysteroid (17-beta) dehydrogenase 6 homolog | NM_003725 | 84 |
| CDH2 | N-cadherin (neuronal) | NM_001792 | 78-96 |
| CDH11 | OB-cadherin (osteoblast) | NM_001797 | 63-96 |
| PCA3 | prostate cancer gene 3 | AF103907 | 80-103 |
| PKP1 | Plakophilin 1 | NM_000299 | 71-86 |
| KLK3 | prostate specific antigen | NM_001030047 | 64-83 |
| PTPR | protein tyrosine phosphatase, receptor type, f polypeptide | NM_003625 | 66 |
| RET | ret proto-oncogene | NM_020975 | 90-97 |
| RORB | RAR-related orphan receptor B | NM_006914 | 66 |
| RRM2 | ribonucleotide reductase M2 | NM_001034 | 79 |
| SFRP2 | secreted frizzled-related protein 2 | NM_003013 | 129 |
| SGP28 | specific granule protein (28 kDa)/cysteine-rich secretory protein 3 CRISP3 | NM_006061 | 111 |
| SNAI2 | snail homolog 2 SNAI2 | NM_003068 | 79-86 |
| SNAI1 | snail homolog 1 Snail | NM_005985 | 66 |
| SPINK1 | serine peptidase inhibitor, Kazal type 1 | NM_003122 | 85 |
| TGM4 | transglutaminase 4 (prostate) | NM_003241 | 87-97 |
| TMPRSS2 | transmembrane protease, serine 2 | NM_005656 | 112 |
| TWIST | twist homolog 1 | NM_000474 | 115 |

Prostate cancer specimens in the following categories were used:
- Low grade prostate cancer (LG): tissue specimens from primary tumors with a Gleason Score ≤6 obtained after radical prostatectomy. This group represents patients with a good prognosis.
- High grade prostate cancer (HG): tissue specimens from primary tumors with a Gleason Score ≥7 obtained after radical prostatectomy. This group represents patients with poor prognosis.

- Prostate cancer metastases: tissue specimens are obtained from positive lymfnodes after LND or after autopsy. This group represents patients with poor prognosis
- Castration resistant prostate cancer (CRPC): tissue specimens are obtained from patients that are progressive under endocrine therapy and who underwent a transurethral resection of the prostate (TURP).
All tissue samples were snap frozen and cryostat sections were stained with hematoxylin and eosin (H.E.). These H.E.-stained sections were classified by a pathologist.

Tumor areas were dissected. RNA was extracted from 10 µm thick serial sections that were collected from each tissue specimen at several levels. Tissue was evaluated by HE-staining of sections at each level and verified microscopically. Total RNA was extracted with TRIzol® (Invitrogen, Carlsbad, CA, USA) according to the manufacturer's instructions. Total RNA was purified using the RNeasy mini kit (Qiagen, Valencia, CA, USA). RNA quantity and quality were assessed on a NanoDrop 1000 spectrophotometer (NanoDrop Technologies, Wilmington, DE, USA) and on an Agilent 2100 Bioanalyzer (Agilent Technologies Inc., Santa Clara, CA, USA).

Two µg DNase-treated total RNA was reverse transcribed using superscript™ II Reverse Transcriptase (Invitrogen) in a 37.5 µl reaction according to the manufacturer's protocol. Reactions were incubated for 10 minutes at 25°C, 60 minutes at 42°C and 15 minutes at 70°C. To the cDNA, 62.5 µl milliQ was added.

Gene expression levels were measured using the TaqMan® Low Density Arrays (TLDA; Applied Biosystems). A list of assays used in this study is given in **Table 5.** Of the individual cDNAs, 3 µl is added to 50 µl Taqman® Universal Probe Master Mix (Applied Biosystems)and 47 µl milliQ. One hundred µl of each sample was loaded into 1 sample reservoir of a TaqMan® Array (384-Well Micro Fluidic Card) (Applied Biosystems). The TaqMan® Array was centrifuged twice for 1 minute at 280g and sealed to prevent well-to-well contamination. The cards were placed in the micro-fluid card sample block of an 7900 HT Fast Real-Time PCR System (Applied Biosystems). The thermal cycle conditions were: 2 minutes 50°C, 10 minutes at 94.5°C, followed by 40 cycles for 30 seconds at 97°C and 1 minute at 59.7°C.

Raw data were recorded with the Sequence detection System (SDS) software of the instruments. Micro Fluidic Cards were analyzed with RQ documents and the RQ Manager Software for automated data analysis. Delta cycle threshold (Ct) values were determined as the difference between the Ct of each test gene and the Ct of hypoxanthine phosphoribosyltransferase 1 (HPRT) (endogenous control gene). Furthermore, gene expression values were calculated based on the comparative threshold cycle (Ct) method, in which a normal prostate RNA sample was designated as a calibrator to which the other samples were compared.

For the validation of the differentially expressed genes found by the GeneChip® Human Exon 1.0 ST Array, 70 prostate cancer specimen were used in TaqMan® Low Density arrays (TLDAs). In these TLDAs, expression levels were determined for the 33 genes of interest. The prostate cancer specimens were put in order from low Gleason scores, high Gleason scores, CRPC and finally prostate cancer metastasis. Both GeneChip® Human Exon 1.0 ST Array and TLDA data were analyzed using scatter- and box plots.

In the first approach, scatterplots were made in which the specimens were put in order from low Gleason scores, high Gleason scores, CRPC and finally prostate cancer metastasis. In the second approach, clinical follow-up data were included. The specimens were categorized into six groups: prostate cancer patients with curative treatment, patients with slow biochemical recurrence (after 5 years or more), patients with fast biochemical recurrence (within 3 years), patients that became progressive, patients with CRPC and finally patients with prostate cancer metastasis. After analysis of the box- and scatterplots using both approaches, a list of suitable genes indicative for prostate cancer and the prognosis thereof was obtained (**Table 7,** **Figures 34-40****).**

**Table 7: List of genes identified**

| **Symbol** | **Gene description** | **Accession number** | **Amplicon size** |
|---|---|---|---|
| HOXC6 | homeobox C6 | NM_004503 | 87 |
| SFRP2 | secreted frizzled-related protein 2 | NM_003013 | 129 |
| HOXD10 | homeobox D10 | NM_002148 | 61 |
| RORB | RAR-related orphan receptor B | NM_006914 | 66 |
| RRM2 | ribonucleotide reductase M2 | NM_001034 | 79 |
| TGM4 | transglutaminase 4 (prostate) | NM_003241 | 87-97 |
| SNAI2 | snail homolog 2 SNAI2 | NM_003068 | 79-86 |

HOXC6 (**Figure 34**): The present GeneChip® Human Exon 1.0 ST Array data showed that HOXC6 was upregulated in prostate cancer metastases compared with primary high and low grade prostate cancers. Validation experiments using TaqMan® Low Density arrays confirmed this upregulation. Furthermore, HOXC6 was found to be upregulated in all four groups of prostate cancer compared with normal prostate. Therefore, HOXC6 has diagnostic potential.

Using clinical follow-up data, it was observed that all patients with progressive disease and 50% of patients with biochemical recurrence within 3 years after initial therapy had a higher upregulation of HOXC6 expression compared with patients who had biochemical recurrence after 5 years and patients with curative treatment. The patients with biochemical recurrence within 3 years after initial therapy who had higher HOXC6 expression also had a worse prognosis compared with patients with lower HOXC6 expression. Therefore, HOXC6 expression is correlated with prostate cancer progression.

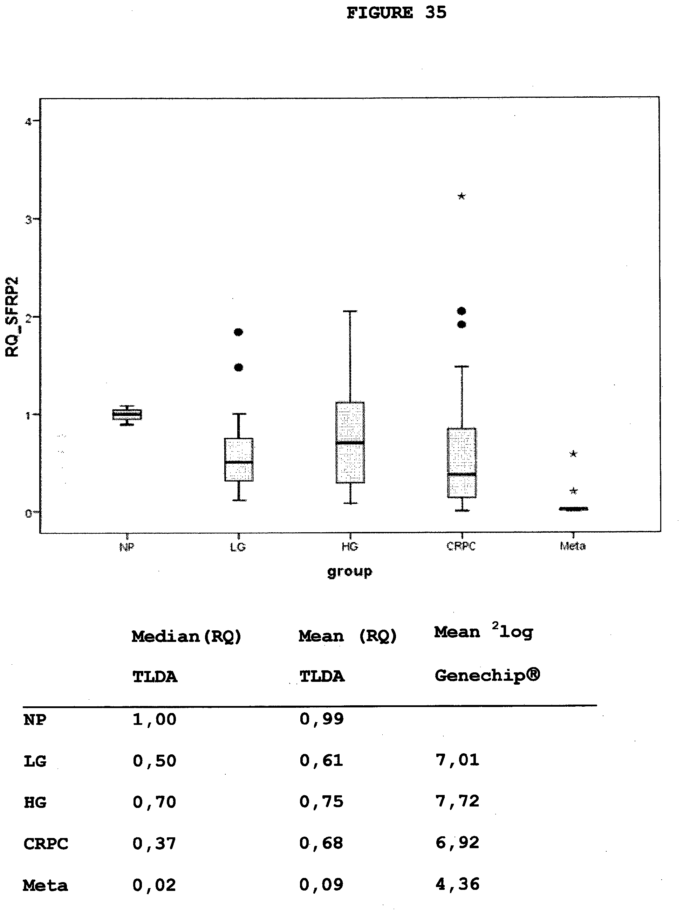

SFRP2 (**Figure 35**): The present GeneChip® Human Exon 1.0 ST Array data showed that SFPR2 was downregulated in prostate cancer metastases compared with primary high and low grade prostate cancers. Validation experiments using TaqMan® Low Density arrays confirmed this downregulation. Furthermore, SFRP2 was found to be downregulated in all four groups of prostate cancer compared with normal prostate. Therefore, SFRP2 has diagnostic potential.

Using clinical follow-up data, differences were observed in SFRP2 expression between the patients with curative treatment, biochemical recurrence after initial therapy and progressive disease. More than 50% of metastases showed a large downregulation of SFRP2. Moreover, also a few CRPC patients showed a very low SFRP2 expression. Therefore, SFRP2 can be used for the detection of patients with progression under endocrine therapy (CRPC) and patients with prostate cancer metastasis. It is therefore suggested, that in combination with a marker that is upregulated in metastases, a ratio of that marker and SFRP2 could be used for the detection of circulating tumor cells.

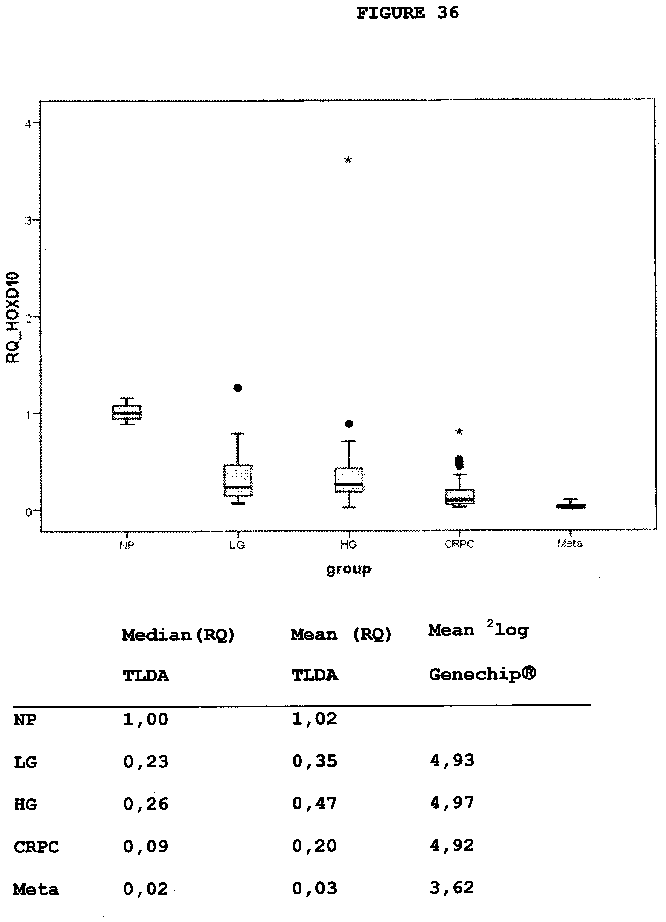

HOXD10 (**Figure 36**): The present GeneChip® Human Exon 1.0 ST Array data showed that HOXD10 was downregulated in prostate cancer metastases compared with primary high and low grade prostate cancers. Validation experiments using TaqMan® Low Density arrays confirmed this downregulation. Furthermore, HOXD10 was found to be downregulated in all four groups of prostate cancer compared with normal prostate. Therefore, HOXD10 has diagnostic potential.

Using clinical follow-up data, differences were observed in HOXD10 expression between the patients with curative treatment, biochemical recurrence after initial therapy and progressive disease. All metastases showed a large downregulation of HOXD10. Moreover, also a few CRPC patients showed a low HOXD10 expression. Therefore, HOXD10 can be used for the detection of patients with progression under endocrine therapy (CRPC) and patients with prostate cancer metastases.

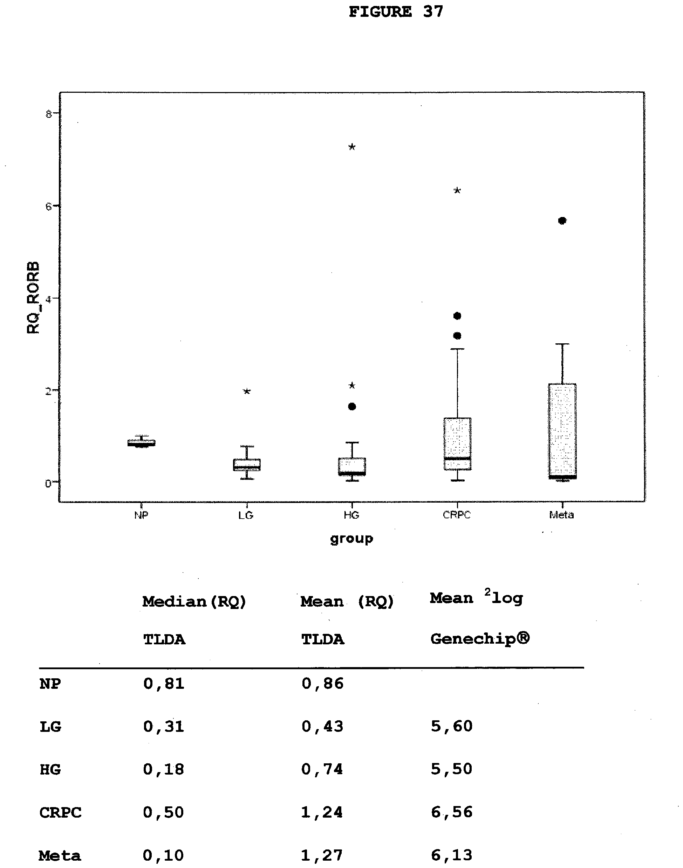

RORB (**Figure 37**): The present GeneChip® Human Exon 1.0 ST Array data showed that RORB was upregulated in prostate cancer metastases and CRPC compared with primary high and low grade prostate cancers. Validation experiments using TaqMan® Low Density arrays confirmed this upregulation. Furthermore, RORB was found to be downregulated in all low and high grade prostate cancers compared with normal prostate. In CRPC and metastases RORB is re-expressed at the level of normal prostate. Therefore, RORB has diagnostic potential.

Using clinical follow-up data, differences were observed in RORB expression between the patients with curative treatment, biochemical recurrence after initial therapy and progressive disease. However, in a number of cases in the CRPC and metastases the upregulation of RORB coincides with a downregulation of SFRP2. Using a ratio of RORB over SFRP2 could detect 75% of prostate cancer metastases. Furthermore, a number of CRPC patients had a high RORB/SFRP2 ratio. Therefore, this ratio can be used in the detection of patients with circulating tumor cells and progressive patients under CRPC.

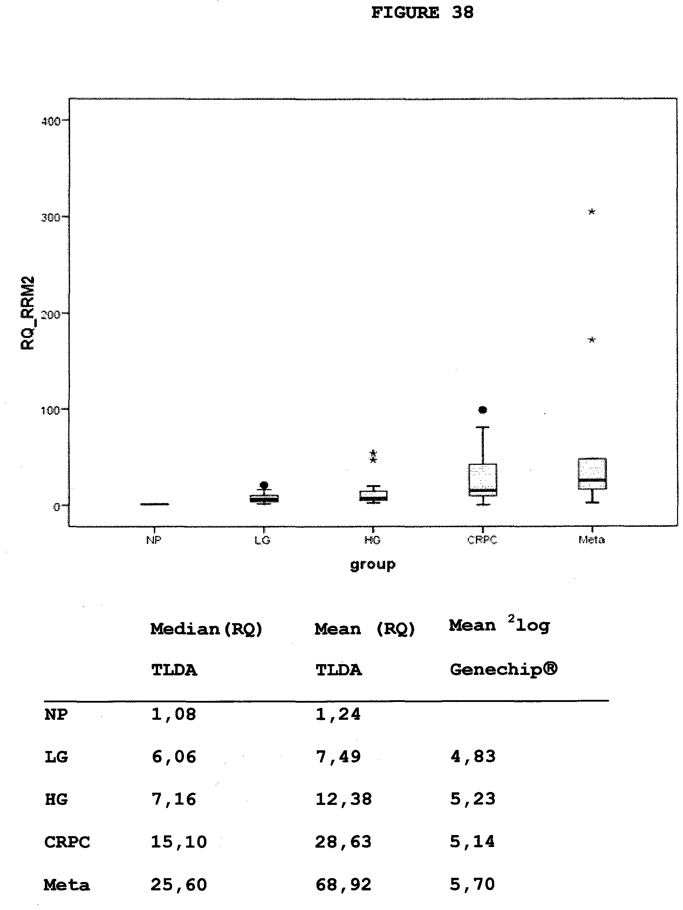

RRM2 (**Figure 38**): Experiments using TaqMan® Low Density arrays showed upregulation of RRM2 in all four groups of prostate cancer compared with normal prostate. Therefore, RRM2 has diagnostic potential. Moreover, the expression of RRM2 is higher in CRPC and metastasis showing that it may be involved in the invasive and metastatic potential of prostate cancer cells. Therefore, RRM2 can be used for the detection of circulating prostate tumor cells.

Using clinical follow-up data, differences were observed in RRM2 expression between the patients with curative treatment, biochemical recurrence after initial therapy and progressive disease.

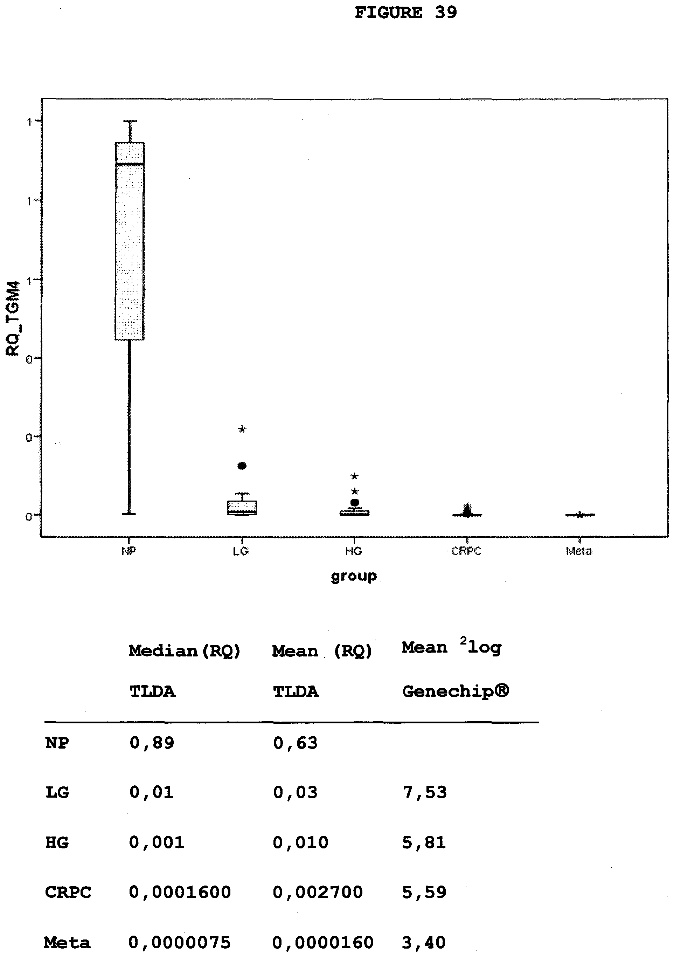

TGM4 (**Figure 39**): The present GeneChip® Human Exon 1.0 ST Array data showed that TGM4 was downregulated in prostate cancer metastases compared with primary high and low grade prostate cancers. Validation experiments using TaqMan® Low Density arrays confirmed this downregulation. Furthermore, TGM4 was found to be extremely downregulated in all four groups of prostate cancer compared with normal prostate. Therefore, TGM4 has diagnostic potential.

Using clinical follow-up data, it was observed that patients with progressive disease showed a stronger downregulation of TGM4 (subgroup of patients) compared with patients with curative treatment and biochemical recurrence after initial therapy. In metastases the TGM4 expression is completely downregulated. Therefore, TGM4 has prognostic potential.

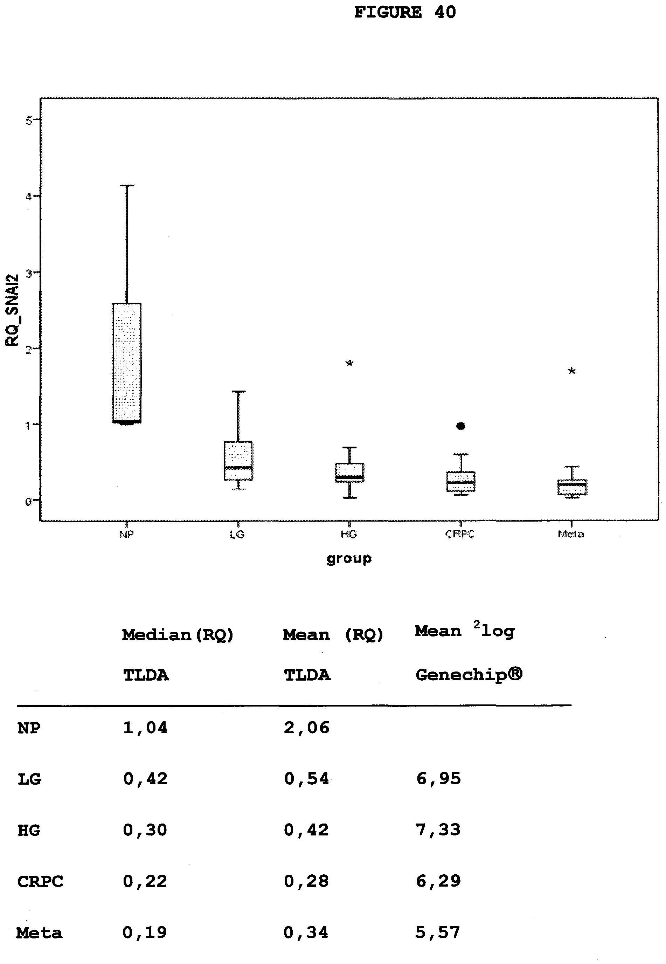

SNAI2 (**Figure 40**): The present GeneChip® Human Exon 1.0 ST Array data showed that SNAI2 was downregulated in prostate cancer metastases compared with primary high and low grade prostate cancers. Validation experiments using TaqMan® Low Density arrays confirmed this downregulation. Furthermore, SNAI2 was found to be downregulated in all four groups of prostate cancer compared with normal prostate. Therefore, SNAI2 has diagnostic potential.

Using clinical follow-up data, differences were observed in SNAI2 expression between the patients with curative treatment, biochemical recurrence after initial therapy and progressive disease.

## Claims

1. Method for in vitro diagnosing high grade PrCa (HG PrCa), PrCa Met and CRPC prostate cancer in a human individual comprising:
- determining the expression of DLX1; and
- establishing up or down regulation of expression of DLX1 as compared to expression of DLX1 in a sample from an individual without prostate cancer;
thereby providing said *in vitro* diagnosis of high grade PrCa (HG PrCa), PrCa Met and CRPC prostate cancer.

2. Method according to claim 1, wherein determining said expression comprises determining mRNA expression.

3. Use of DLX1 expression for in vitro diagnosing high grade PrCa (HG PrCa), PrCa Met and CRPC prostate cancer, preferably CRPC.

## Patentansprüche

1. Verfahren für die In-vitro-Diagnose eines hochgradigen PrCa- (HG-PrCa-), PrCa-Met- und CRPC-Prostatakarzinoms in einem menschlichen Individuum, das aufweist:
- Bestimmen der Expression von DLX1; und
- Erzeugen einer Herauf- oder Herabregulierung der Expression von DLX1 im Vergleich zu der Expression von DLX1 in einer Probe von einem Individuum ohne Prostatakarzinom;
wodurch die In-vitro-Diagnose des hochgradigen PrCa- (HG-PrCa-), PrCa-Met- und CRPC-Prostatakarzinoms bereitgestellt wird.

2. Verfahren nach Anspruch 1, wobei die Bestimmung der genannten Expression die Bestimmung der mRNA-Expression aufweist.

3. Verwendung der DLX1-Expression für die In-vitro-Diagnose eines hochgradigen PrCa- (HG-PrCa-), PrCa-Met- und CRPC-Prostatakarzinoms, vorzugsweise CRPC.

## Revendications

1. Procédé de diagnostic *in vitro* du cancer de la prostate PrCa de haut grade (HG PrCa), PrCa Met et CRPC chez un individu humain comprenant :
- la détermination de l'expression de DLX1 ; et
- l'établissement d'une régulation positive ou négative de l'expression de DLX1 par comparaison à l'expression de DLX1 dans un échantillon d'un individu ne présentant pas de cancer de la prostate ;
en établissant ainsi ledit diagnostic *in vitro* du cancer de la prostate PrCa de haut grade (HG PrCa), PrCa Met et CRPC.

2. Procédé selon la revendication 1, dans lequel la détermination de ladite expression comprend la détermination de l'expression de l'ARNm.

3. Utilisation de l'expression de DLX1 pour le diagnostic *in vitro* du cancer de la prostate PrCa de haut grade (HG PrCa), PrCa Met et CRPC, de préférence le CRPC.
